# EUROPEAN PATENT SPECIFICATION

(11) **EP 0 918 496 B1**
(45) Date of publication and mention of the grant of the patent: **14.06.2000**
(21) Application number: 97908050.4
(22) Date of filing: 07.03.1997
(51) Int. Cl.: A61F 2/06

(54) **ENDOLUMINAL PROSTHESES FOR MULTIPLE-BRANCH BODY LUMEN SYSTEMS**
ENDOLUMINALE PROTHESEN FÜR MEHRFACHABZWEIGUNGEN AUFWEISENDE KÖRPERLUMENSYSTEME
PROTHESE ENDOLUMINALE POUR DES ORGANES DU CORPS HUMAIN A PLUSIEURS LUMIERES ET RAMEAUX

(30) Priority: 13.03.1996 US 615697; 07.10.1996 US 28928 P
(43) Date of publication of application: 02.06.1999
(73) Proprietor: MEDTRONIC, INC., Minneapolis, Minnesota 55432 (US)
(72) Inventor: COX, Brian, Cupertino, CA 95104 (US); EVANS, Michael, A., Palo Alto, CA 94301 (US); WILL, Allan, Atherton, CA 94027 (US); LENKER, Jay, A., Los Altos Hills, CA 94024 (US); FREISLINGER, Kirsten, Menlo Park, CA 94025 (US); FOGARTY, Thomas, J., Portola Valley, CA 94028 (US)
(74) Representative: Kazi, Ilya
(86) International application number: US9703545
(87) International publication number: WO9733532

(56) References cited:
- EP-A- 0 357 003
- EP-A- 0 646 365
- WO-A-95/09586
- WO-A-95/18585
- WO-A-95/23563

## Description

### BACKGROUND OF THE INVENTION

### 1. Field of the Invention

The present invention relates generally to tubular prostheses, such as grafts, stents, stent-grafts, and the like. More particularly, the present invention provides radially expandable tubular prosthetic structures which are deployable within tortuous body lumens, particularly within branching blood vessels for the treatment of abdominal and other aneurysms.

Vascular aneurysms are the result of abnormal dilation of a blood vessel, usually resulting from disease and/or genetic predisposition, which can weaken the arterial wall and allow it to expand. While aneurysms can occur in any blood vessel, most occur in the aorta and peripheral arteries, with the majority of aortic aneurysms occurring in the abdominal aorta, usually beginning below the renal arteries and often extending into one or both of the iliac arteries.

Aortic aneurysms are most commonly treated in open surgical procedures, where the diseased vessel segment is bypassed and repaired with an artificial vascular graft. While considered to be an effective surgical technique, particularly considering the alternative of a usually fatal ruptured abdominal aortic aneurysm, conventional vascular graft surgery suffers from a number of disadvantages. The surgical procedure is complex and requires experienced surgeons and well equipped surgical facilities. Even with the best surgeons and equipment, however, patients being treated frequently are elderly and weakened from cardiovascular and other diseases, reducing the number of eligible patients. Even for eligible patients prior to rupture, conventional aneurysm repair has a relatively high morality rate, usually from 2% to 10%. Morbidity related to the conventional surgery includes myocardial infarction, renal failure, impotence, paralysis, and other conditions. Additionally, even with successful surgery, recovery takes several weeks, and often requires a lengthy hospital stay.

In order to overcome some or all of these drawbacks, endovascular prosthesis placement for the treatment of aneurysms has been proposed. Although very promising, many of the proposed methods and apparatus suffer from undesirable limitations. In particular, proper matching of an endovascular prosthesis with the complex and highly variable vascular geometry can be problematic.

Proper matching of the prosthesis to the proximal neck of the aortic vessel and the branching blood vessels is critical to the treatment of an aneurysm. The prosthesis preferably extends axially beyond the weakened portion of the blood vessel to anchor securely in the less diseased vessel wall. To prevent the leakage of blood through a ruptured aneurysm, and also to prevent the release of thrombus from within the distended aneurysm and into the bloodstream, it is also preferable that the prosthetic lumen be substantially sealed against the healthy endolithium. The prosthetic lumen should remain open despite physiological movement of the vasculature and environmental stresses, so as to promote the free flea of blood. Furthermore, the geometry of the prosthetic lumen at the luminal intersection where the abdominal aorta meets the iliac arteries is of particular importance, as this bifurcation can have a significant impact on the relative blood flows through the two iliac arteries.

Unfortunately, the size, extent, and specific geometry of abdominal aortic aneurysms can vary widely from patient to patient. While the aneurysm is often downstream of the renal arteries, as noted above, it may begin in very close proximity to these lateral branching blood vessels, and in some cases will extend up to, above, and along the renals themselves. Additionally, while the aneurysm itself is typically a distension of the vessel wall, the path the prosthesis must follow within the diseased vessel may be fairly convoluted. For example, the abdominal aorta typically defines a significant bend between the renal arteries and the iliac arch when viewed from a lateral position. This aortic bend often remains quite pronounced despite the presence of the distended aneurysm, and complicates the sealing and anchoring of the endoluminal prosthesis adjacent the renal arteries.

Abdominal aortic aneurysms also appear to have a significant effect on the geometry of the intersection between the abdominal aorta and iliac arteries. Even among healthy patients, there are significant variations in the angles defined by the iliac arteries relative to the aorta, typically being anywhere in the range between 15-45°. The variation in aorta iliac angularity is often much wider in patients seeking therapy for aneurysms. In fact, iliac arteries which branch off from an aorta with a local angle of over 90° have been found in aneurysm patients.

Known branching endoluminal prostheses are generally formed as tubular, radially expandable stent-grafts. In contrast with the convoluted branchings of diseased body lumens, these stent-graft structures have typically been formed with simplistic cylindrical frames or "stents." A separate liner or "graft" is typically attached to the frame to prevent blood flow through a ruptured vessel wall. Such liners are often formed from inelastic fabrics to prevent pressure from distending a weakened luminal wall. Typically, these branching structures are primarily supported from immediately below the renal arteries. Patients may not be eligible for these known endovascular aneurysm therapies if this portion of the aorta is weakened by disease.

The branching stent-graft structures of the prior art have generally comprised uniform structures, in which the smaller iliac branch portions form cylinders which are substantially parallel to the aortic portion when the prosthesis is at rest. Although these straight branching prostheses are intended to deform somewhat to accommodate the branch angles of body lumen systems, the imposition of substantial axial bends on known endovascular stent-grafts tends to cause folding, kinking, or wrinkling which occludes their lumens and degrades their therapeutic value. Still another disadvantage of known bifurcated stent-grafts is that even when they are flexed to accommodate varying branch geometry, the prosthetic bifurcation becomes distorted, creating an unbalanced flow to the branches. To overcome these limitations, it has often been necessary to limit these highly advantageous, minimally invasive endovascular therapies to patients having vascular geometries and abdominal aortic aneurysms which fall within very narrow guidelines.

For these reasons, it would be desirable to provide improved endoluminal prostheses. It would further be desirable to provide improved branching endoluminal prostheses. It would be particularly desirable to provide endoluminal prostheses which would accommodate widely varying lumen system geometries, and which would thereby increase the proportion of patients eligible to receive these highly advantageous endoluminal prosthetic therapies for treatment of abdominal aortic aneurysms and other disease conditions of the body lumen systems.

### 2. Description of the Background Art

Co-pending U.S. Patent Application Serial No. 08/538,706, filed October 3, 1995, describes modular prostheses and construction methods.

WO-A-9 509 586 describes a branching endoluminal prosthesis having a radially expandable trunk portion and radially expandable first and second branch portions with first and second prosthetic branch lumens.

Patent Application Serial No. 08/628,797, filed April 5, 1996, describes advantageous radiopaque marker structures and patterns for *in situ* assembly of endoluminal prostheses.

U.S. Patent No. 5,064,435 describes a self-expanding prosthesis which maintains a stable axial length during radial expansion by anchoring of radial outward flares at each end, and by sliding of an overlapping medial region therebetween. U.S. Patent No. 5,211,658 describes a method and device for endovascular repair of an aneurysm which makes use of separately introduced frame and liner structures. A similar method of repairing blood vessels is described in U.S. Patent No. 5,078,726, in which a locking stent is expanded within a vascular graft which has been positioned within the blood vessel. The *in situ* deployment of an aortic intraluminal prosthesis by a catheter having two inflatable balloons is described in U.S. Patent No. 5,219,355.

European patent application publication no. 0 551 179 describes a method for deploying two tubular grafts which extend in parallel from the renals and into the aorta. U.S. Patent No. 5,360,443 describes a bifurcated aortic graft which is secured to the aorta by a plastically deformable frame positioned between the renal arteries and the iliaci. Soviet Patent 145-921 describes a bifurcated blood vessel prosthesis having a fastening element which extends past the renal arteries to prevent migration. U.S. Patent No. 4,774,949 describes a catheter having a lumen adapted to access branch arteries.

U.S. Patent Application Nos. 4,550,447 and 4,647,416 describe vascular PTFE grafts which include transverse ribs integral with a tube wall, and methods for their production. U.S. Patent Application No. 5,443,499 describes a radially expandable tubular prostheses for intraluminal implantation within children. U.S. Patent Application Nos. 5,229,045 and 5,387,621 describe porous membranes based on unstable polymer solutions which are suitable for vascular prostheses, and methods for their production.

### SUMMARY OF THE INVENTION

The present invention provides a branching intraluminal prostheses for use in a branching body lumen system that includes a trunk lumen and first and second branch lumens. The prostheses comprises a radially expandable tubular trunk portion having a prosthetic trunk lumen, and radially expandable tubular first and second branch portions with first and second prosthetic branch lumens, respectively. A radially expandable tubular lumen separation portion provides fluid communication between the prosthetic trunk lumen and the first and second prosthetic branch lumens. The expanded trunk portion is more axially flexible than the lumen separation portion.

Although it is often considered desirable to maximize the column strength of endoluminal prostheses, and although the trunk portion will generally have a larger cross-section than much of the remainder of a branching endoluminal prostheses, in connection with the present invention it has been found that insufficient flexibility along the trunk portion may result in leakage between a bifurcated prosthesis and the trunk lumen of the body lumen system. Specifically, leaks will be produced between known uniform bifurcated prostheses and the dorsal bend which is typically found immediately downstream of the renal arteries along the abdominal aorta. On the other hand, the lumen separation portion benefits from a less axially flexible structure to avoid distortion of the flow balance between the luminal branches when conforming the prosthetic geometry to a torturous body lumen system. The present invention therefore provides non-uniform prosthetic structures which are locally optimized to meet these contradictory requirements.

In some embodiments, a trunk sealing cuff is provided opposite the Y-connector to seal between the prosthetic trunk lumen and the trunk lumen of the body lumen system. Similarly, the first and second branch portions are also more axially flexible than the lumen separation portion, and ideally include branch sealing cuffs opposite the lumen separation. These sealing cuffs may also benefit from relatively stiff structures, particularly where they help to anchor the prosthesis within the body lumen. The resulting prosthetic structure separates the luminal sealing, the axial conforming, and the flow separating functions of the branching prostheses to distinct axial portions of the prosthetic structure, allowing these portions to be still further independently optimized.

Preferably, the present invention provides an endoluminal prosthesis comprising a radially expandable tubular liner having a lumen which defines an axis. A helical coil supports the liner, the coil defining a plurality of loops which are separated to enhance the axial flexibility of the prosthesis. The helical coil elongates during expansion of the liner to avoid unwinding of the coil relative to the liner. Hence, the coil may be attached at a plurality of attachment points along the length of the coil.

Preferably, the present invention provides a bifurcated prosthesis comprising a liner supported by a plurality of ring frames. The tubular liner has a trunk end, a branch end, and an axis therebetween, and defines first and second lumens. The lumens are adjacent to each other at their trunk ends. The ring frames are independently attached axially along the liner to hold the first and second lumens open, and to seal liner the trunk end of the liner against a surrounding body lumen. Such a structure provides both bifurcated lumen patency and flexibility in an integrated structure.

### BRIEF DESCRIPTION OF THE DRAWINGS

Fig. 1 is a side view of an exemplary cylindrical vascular stent-graft having axially constant characteristics.

Fig. 2 is a perspective view of an exemplary delivery catheter for use with the prostheses of the present invention, with a portion of the distal end broken away to disclose a prostheses therein.

Figs. 3A-3C illustrate a bifurcated endovascular prosthesis having a relatively rigid expanded Y-connector portion, axially flexible branch and trunk portions, and sealing/anchoring cuffs, according to the principles of the present invention.

Fig. 4 illustrates a prostheses having two stent-graft portions connected by a flexible joint comprising an integrally ribbed polymer tube.

Figs. 5A-5D illustrate an endoluminal prosthetic structure in which a frame is supported by a helical coil of expansible diamond shaped elements, for use in the flexible portions of the prosthesis of Figs. 3A-3C.

Fig. 5E illustrates a method for making an endoluminal prosthesis having a helical coil by first attaching the coil material to a strip of liner material, winding the liner strip over a mandrel, and sewing the strip in a helical shape.

Figs. 5F-5H illustrates alternative stent-graft sealing structures.

Figs. 6A-6C illustrate alternative flexible prosthetic structures in which the liner is supported by a plurality of cylindrical segments.

Fig. 7A illustrates an endoluminal prosthetic structure in which a liner is supported by a plurality of self-expanding loops, and in which serpentine malleable connectors extend between adjacent loops.

Figs. 7B-7G show alternative connector structures and connector attachment mechanisms for use in the prosthesis of Fig. 7A.

Figs. 8A-8F illustrate a method for deploying a self-supporting endoluminal hub module within a luminal intersection.

Figs. 9A-9B illustrate alternative endoluminal hub modules having flexible portions between their trunk and branch portions.

Figs. 10A-10C illustrate a method for positioning guide wires adjacent to a luminal intersection to promote precise positioning of an endoluminal prostheses by selectively tensioning opposed guide wire ends.

Figs. 11A-11C illustrate a method for deploying a branching endoluminal prostheses by first deploying a branch module which extends across the trunk lumen and extending into opposing branch lumens, and by then deploying a trunk module within a trunk port of the branch module.

Fig. 12 illustrates an alternative branching endoluminal prostheses in which a branch module is positioned through a deployed trunk module.

Fig. 13 illustrates an alternative branching inner luminal prostheses in which independent branch modules are deployed within an expanded trunk module.

Figs. 14A-14B illustrate a method for deploying a branching endoluminal prostheses in which a spacer module is first deployed to provide support for the trunk module from adjacent to the branch lumens of the body lumen system.

Figs. 15A-15B illustrate a method for deploying a branching prostheses in which a tapering primary module is first deployed adjacent a luminal intersection.

Figs. 16A-16B illustrate a still further alternative method for deploying a branching endoluminal prostheses in which the trunk module is deployed within and supported by a previously deployed branch module.

Figs. 17A-17D illustrate an alternative branching endoluminal prostheses in which at least one branch portion is compressed within the trunk portion during positioning and deployment.

Figs. 18A-18B illustrate alternative branching endoluminal prosthetic structures having reduced compressed frame volumes and adjustable branch lengths.

Fig. 19 illustrates a branching endoluminal prosthesis having a short trunk portion to increase overall axial flexibility, according to the principles of the present invention.

Fig. 20 illustrates an endoluminal prosthetic system having first and second branching prosthetic modules.

Fig. 21 illustrates an assembled endoluminal prosthetic system having two branching modules which overlap within a predetermined range to accommodate variations in the lengths of branches within a body lumen system.

Fig. 22 illustrates a modular endoluminal prosthetic therapy for aortic aneurysms which extend along at least one aortic artery to a hypogastric artery.

Fig. 23 illustrates a bifurcated prosthetic system structure in which a plurality of ring frames independently support the bifurcated liner to provide both lumenal patency and axial flexibility, and in which the branch portions are axially conformable.

Figs 24A-C illustrate the cross-sections of the independent ring frames of the bifurcated prosthetic module of Fig. 23

Fig. 25 illustrates deployment of the prosthetic system of Fig. 23.

Fig. 26 schematically illustrates differences in distances between the renal and hypogastric arteries, as can be accommodated by varying the modular overlap and/or with axial conformability.

Fig 27 illustrates a bifurcated module with enhanced axial variability by minimizing the overlap variability, according to the principles of the present invention.

### DETAILED DESCRIPTION OF THE SPECIFIC EMBODIMENTS

The present invention provides radially expansible tubular prostheses, particularly grafts, stents, and stent-grafts, which are highly adaptable to varying luminal system geometries. The prostheses of the present invention are suitable for a wide variety of therapeutic uses, including stenting of the ureter, urethra, trachea, branchi, esophagus, biliary tract, and the like. The present devices will also be useful for the creating of temporary or long term lumens, such as the formation of fistulas.

The prosthetic structures of the present invention will find their most immediate use as endovascular prostheses for the treatment of diseases of the vasculature, particularly aneurysms, stenoses, and the like, and are especially well suited to the distorted aortal/iliac junction of persons having advanced vascular diseases. These prostheses will generally be radially expansible from a narrow diameter configuration to facilitate introduction into the body lumen, typically during surgical cutdown or percutaneous introduction procedures.

The prosthetic structures described hereinbelow will find use in axially uniform cylindrical prostheses, in preassembled bifurcated prostheses, and as prosthetic modules which are suitable for selective assembly either prior to deployment, or *in situ.* Such selective assembly of prosthetic modules to form a customized endoluminal prosthesis is more fully described in co-pending U.S. Patent Application Serial Nos. 60/008,254 and 08/538,706 (1995).

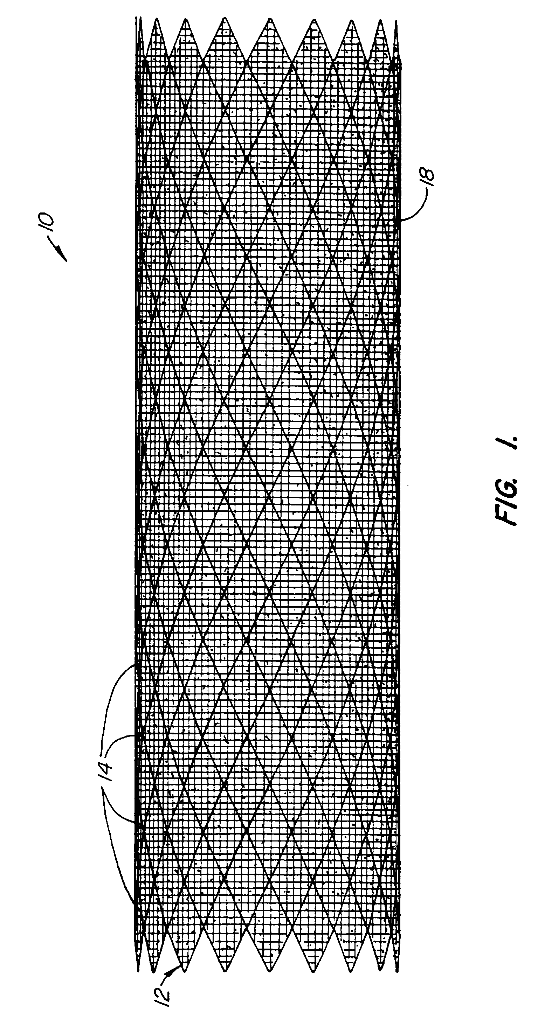

An exemplary cylindrical graft structure 10 is illustrated in Fig. 1. Prostheses 10 comprises a perforate tubular frame 12 which includes a plurality of independent (non-connected) ring frames 14. The tubular frame 12 supports an inner frame 18. Optionally, an outer liner is disposed over the ring frames, either inside of inner liner 18, or in combination therewith.

To secure ring frames 14 in place, and to secure the liner to the perforate tubular frame 12, the liner is typically sutured to the frame. A wide variety of alternative liner/frame attachment mechanisms are available, including adhesive bonding, heat welding, ultrasonic welding, and the like. Where inner and outer liners are used, the ring frames may be sandwiched between the liners and held in place by attaching the liners to each other.

The prostheses 10 will typically have a length in the range from about 20 mm to 500 mm, preferably from 50 mm to 200 mm, with a relaxed diameter in the range from about 4 mm to 45 mm, preferably being in the range from about 5 mm to 38 mm. Alternative stent-graft structures are more fully described in Application Serial No. 08/538,706 (1995).

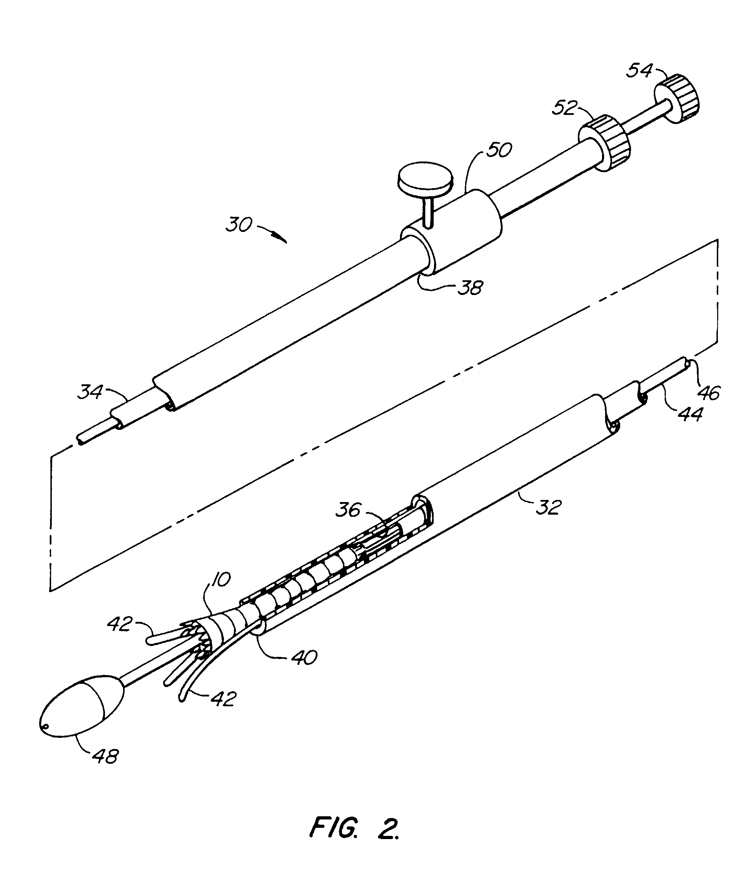

Referring now to Fig. 2, an exemplary delivery catheter 30 for use with the endoluminal prostheses of the present invention comprises a tubular cover 32 and a shaft 34. Cover 32 has a central lumen 36 extending from a proximal end 38 to a distal end 40. Shaft 34 is slidably received within central lumen 36 and extends proximally of cover 32. A plurality of runners 42 extend distally from shaft 34. Runners 42 line a portion of the inner surface of lumen 36, and slide within the lumen of the shaft. Shaft 34 also has a lumen, in which a core shaft 44 is slidably disposed. Core shaft 44 has a guide wire lumen 46. Nosecone 48 is fixed to the distal end of core shaft 44, and can therefore be manipulated independently of runners 42.

Prostheses 10 is radially compressed and restrained within the plurality of runners 42. In turn, cover 32 prevents runners 42 from expanding outward. Runners 42 are formed from a hard material, and distribute the expansion load of prostheses 10 over the inner surface of central lumen 36. The deploying force is applied proximally against a slider 50 attached to a distal end 38 of cover 30, while holding a luer fitting 52 at the distal end of shaft 34, thereby withdrawing the cover proximally from over the prostheses. An additional luer adapter 54 at the distal end of core shaft 44 allows the core shaft to be manipulated independently, and to be releasably secured to the shaft 34. Exemplary methods and devices for placement of the prostheses of the present invention are more fully described in co-pending U.S. Patent Application Serial No. 08/475,200, filed June 7, 1995.

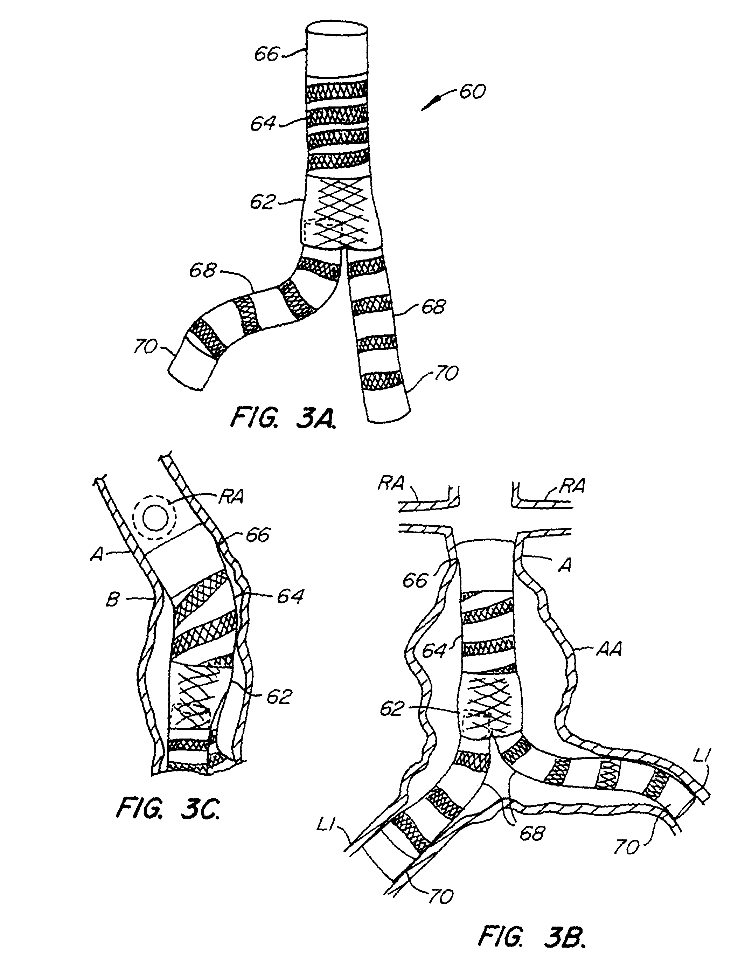

Referring now to Figs. 3A-3C, an exemplary branching endovascular protheses 60 comprises a lumen separation portion 62 between a trunk portion 64 and two branch portions 68. Lumen separation portion 62 preferably comprises a relatively rigid structure, having higher column and hoop strength than the remainder of the prostheses.

In this exemplary embodiment, the lumen separation portion comprises a flexible liner supported by a resiliently expanding frame. The cross-section of the frame adjacent the branches includes discrete lobes which correspond to the first and second branches, and also includes an isthmus therebetween to help prevent an imbalance of flow from the trunk portion to the branch portions. Ideally, the perforate frame of lumen separation portion 62 is continuous along its axial length, increasing the column strength of the lumen separation so that the flow separation geometry of the branching inner lumen remains constant regardless of the flexing of the trunk and/or branch portions.

The advantageous flexibility of branch portions 68 is shown most clearly in Fig. 3B, in which prostheses 60 is shown deployed within an abdominal aorta A downstream of the renal arteries RA, extending beyond an abdominal aortic aneurism AA, and into the right and left iliac arteries RI, LI. Branch portions 68 have relatively high axial flexibility to accommodate the extreme angles between the iliac and abdominal arteries which have been found in patients having such aneurysms.

Trunk sealing cuff 66 and branch sealing cuffs 70 securely anchor the prostheses against the healthy tissue beyond the aneurism, and also seal the prosthetic lumen against the surrounding endolithium of the body lumen system. Trunk sealing cuff 66 will often comprise a polyester such as Dacron™, preferably in an expansible form, ideally as a fabric woven with partially oriented or disoriented polyester fibers in the fill or weave. Alternatively, polyester (or some other fiber) which has been wrapped around a core fiber to allow expansion may be used, or the sealing cuff may comprise a PTFE, silicone, or polyurethane foam to promote sealing between the prosthetic lumen and the surrounding body lumen. Exemplary sealing cuff structures are more fully described in co-pending Patent Application Serial Nos. 08/525,989 and 08/538,706, filed October 3, 1995, and September 8, 1995.

One particular advantage of the axial flexibility of trunk portion 64 can be understood with reference to the lateral view of the abdominal aorta illustrated in Fig. 3C. Although the aneurysm AA generally distends the abdominal aorta, the specific shape and extent of the aneurysm can vary widely. Even when healthy, the abdominal aorta often angles dorsally just downstream of the renal arteries. The presence of this bend B often persists despite the general distension of the abdominal aorta.

Advantageously, flexible trunk portion 64 allows the trunk sealing cuff 66 to anchor securely along the axis of the healthy abdominal aorta adjacent the renal arteries, and greatly helps to reduce perimeter leaks around the upstream end of the trunk portion. Those of skill in the art will understand that the trunk portion would tend to have a relatively high rigidity and column strength, due to its relatively large cross-section (which must accommodate the combined flow for both iliac arteries). It should also be understood that the flexible trunk and leg portions will preferably maintain sufficient hoop strength so that their respective lumens remain open throughout a wide range of branch positions, and despite normal physiological movement and environmental stress from the surrounding body lumen. Hence, the flexible trunk and leg portions will preferable comprise a coiled prosthetic structure or a radially expandable, axially malleable structure as described hereinbelow. Alternatively, the flexible trunk and branch portions may comprise an unsupported (or self-supporting) liner.

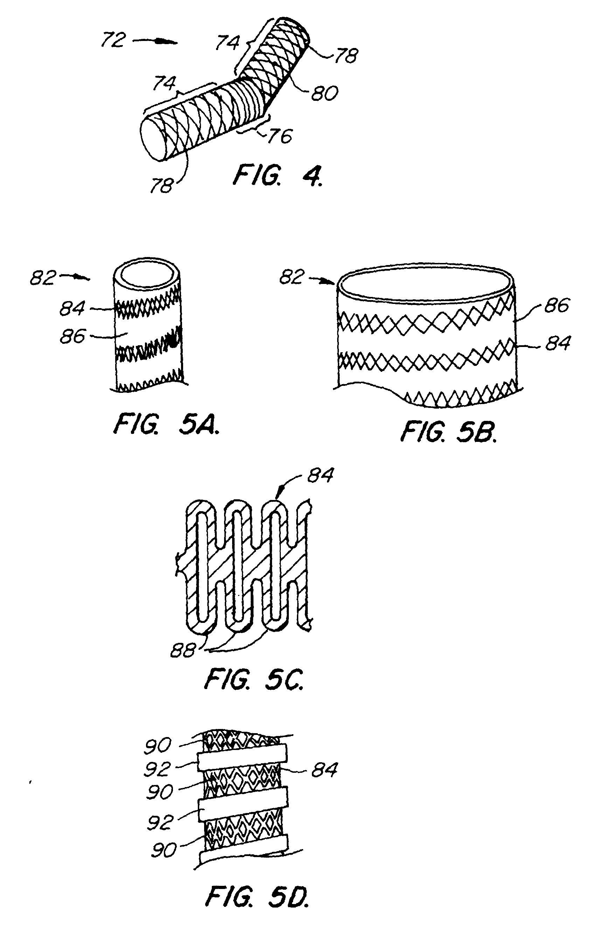

Referring now to Fig. 4, a jointed prosthesis structure 72 provides axial flexibility and kink resistance, and may therefore find use in the flexible sections of exemplary branching endoluminal prosthesis 60 (see Fig. 3A). Jointed prosthesis 72 includes a plurality of stent-graft portions 74 with a joint portion 76 therebetween. Stent-graft portions 74 comprise a liner 80 supported by a perforate radially expandable frame 78. Preferably, joint portion 76 comprises an integrally ribbed polymer tube, as taught by U.S. Patent Nos. 4,647,416 and 4,550,447. Ideally, the joint comprises a ribbed PTFE tube which extends continuously to form the liners of the stent-graft portions.

Advantageously, the framed structure of the stent-graft portion provides the column and hoop strength to support the inner lumen, while the self-supporting joint structure allows the jointed prosthesis to easily adapt to tortuous body lumens. It may be advantageous to provide a series of such jointed stent-graft sections to allow the prosthesis to adapt to the highly tortuous arteries associated with aneurysm patients.

Referring now to Figs. 5A-C, an alternative flexible prosthesis section may comprise a coil-shaped stent structure, in which the coils are separated from each other by a distance which allows the liner material to flex easily, thereby providing a prosthetic structure with both axial flexibility and hoop strength.

A limitation of known coiled expandable stent-graft structures is that they induce relative motion between the liner and support materials, the coil unwinding as the liner expands. Coiled prosthetic structure 82 overcomes this limitation by including an expandable coil 84 with an expansible liner material 86. As the prosthesis perimeter increases in size, expandable coil 84 elongates, preferably by deformation of a series of linked diamond-shaped elements 88. As the coil frame expands with the liner material, these two structures may be attached directly together at a plurality of locations without binding.

The expandable coil may be either self-expanding, preferably comprising a highly resilient material, ideally comprising a shape memory alloy such as super-elastic Nitinol™, or the like. Alternatively, the coil may comprise a malleable material, typically a plastically deformable metal such as stainless steel, tantalum, martensitic shape memory alloy such as Nitinol™, a shape memory polymer, or the like.

The liner will optionally an expandable tubular material, often being a woven polyester such as Dacron™, or may alternatively comprise a plastically expansible material such as PTFE, partially oriented yarn, or an annealed or wrapped composite fiber such as those more fully described in co-pending U.S. Patent Application Serial No. 08/595,944, filed February 6, 1996 (Attorney Docket No. 16380-004010), the full disclosure of which is incorporated herein by reference. Optionally, the liner may also include a ribbed polymer as described above. As illustrated in Fig. 5D, a ribbed PTFE liner will preferably include ribs 92 disposed between the adjacent loops 90 of expandable coil 84.

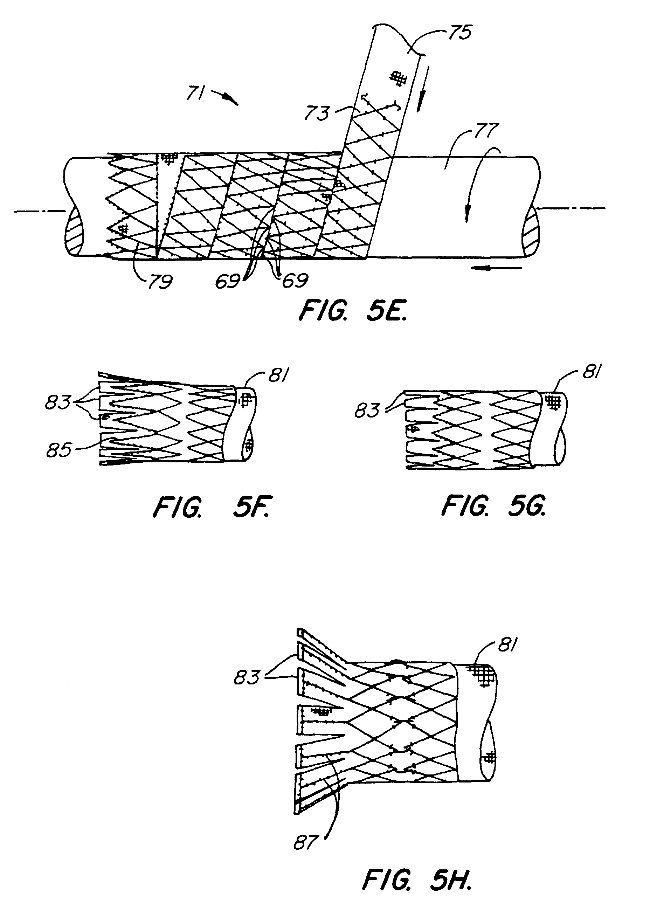

A method of fabricating a helical stent-graft 71 will be described with reference to Fig. 5E. A series of linked diamond-shaped elements 73 are first attached to a strip of liner material 75, typically being stitched with a sewing machine. The ribbon is then wound over a mandrel 77 of the desired size, and adjacent edges of the ribbon are sewn to each other (or otherwise permanently joined). Such a method may be substantially automated and continuous, and is thus particularly beneficial for producing a large number of prostheses. The helical stent-graft may optionally be cut to length, but will preferably include a crown stitched stent-ring 79 for sealing and ends against a surrounding lumen when deployed therein.

A novel feature of helical stent-graft 71 which will have application in a wide range of stent-graft structures is the offsetting of apices 69. Diamond-shaped elements 73 define axially oriented apices 69 at regular intervals along the loops. Through proper sizing of mandrel 77 and monitoring of the loop sewing process, the adjacent apices may optionally be offset from the adjacent apices, each apex ideally being roughly equally spaced from the two adjacent apices as shown. Advantageously, this increases axial flexibility by allowing the liner to flex between loops but without substantially decreasing hoop strength. Conveniently, the column strength may be selectively and locally increased (and axial flexibility correspondingly decreased) by adjusting the winding of ribbon 75 so that the adjacent apices are substantially aligned. In fact, aligned apices may be selectively attached to each other, for example, with a lock stitch pattern (as shown in Fig. 5, 4, and more fully explained in co-pending patent application Serial No. 08/538,706, filed October 3, 1995, to greatly reduce axial flexibility where desired. Clearly such selective offsetting of apices will be effective with ring frames, zig-zag coils, and a wide range of alternative stent-graft structures, and continuous graft configurations.

Alternative sealing structures are illustrated in Figs. 5F-G. Generally, liner 81 is split at one end to form a plurality of sealing flaps 83. Optionally, the sealing flaps are substantially unsupported by the frame. Alternatively, the frame adjacent sealing flaps 83 includes axially elongate members which support the sealing flaps, for example, elongate diamonds 85 or fingers 87. These elongate member (or the sealing flaps themselves) are preferably resiliently biased radially outward, typically by heat setting over a tapered mandrel. In some embodiments, the flaps may fold back along the prosthesis when the prosthesis is compressed for deployment. Regardless, each sealing flap will preferably expand radially outward substantially independently of the other sealing flaps, thereby improving the seal between the end of the prosthesis and a highly irregular body lumen. Optionally more than one row of overlapping sealing flaps may also be used.

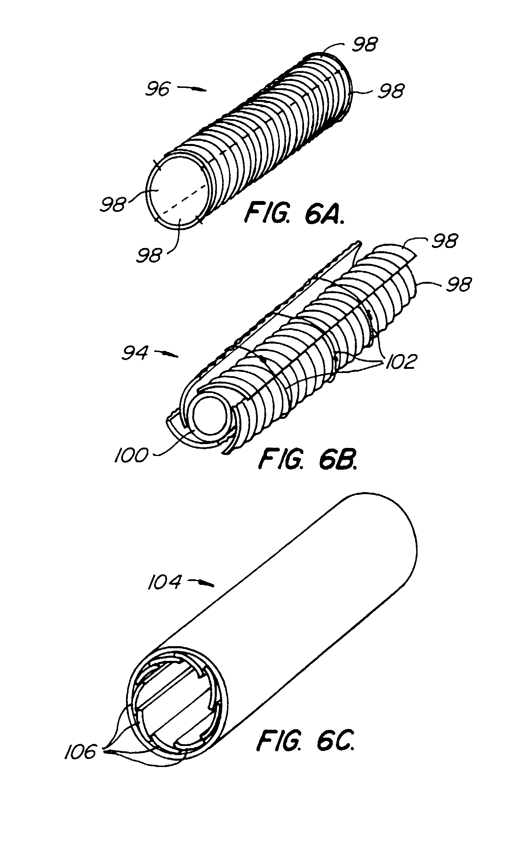

Referring now to Figs. 6A and B, an alternative flexible prosthetic structure may be fabricated by cutting a cylindrical corrugated polyester graft 96 into a series of cylindrical segments. The cylindrical segments may then be used as reinforcing elements by attaching them axially along an expansible tube 100. Suitable expansible tubes may be formed from partially oriented yarn, polypropylene, polyethylene, annealed polyester, PTFE, or the like. The reinforcing elements are preferably free to slide over each other as the liner is expanded *in situ,* and provide some column strength, hoop strength, and kink resistance while also allowing the reinforced lumen to flex axially.

Optionally, a plurality of expansible fibers or yarns 102 could be wrapped around the exterior of the corrugated graft segments to hold the structure in a compact profile, and yet still allow expansion. Alternatively, outer fibers 102 may be frangible, breaking under a predetermined force to allow the prosthesis to be expanded *in situ* to the desired size. An internally supported flexible structure 104 having similar internal reinforcing elements 106 may optionally avoid the use of the external wrapping yarns.

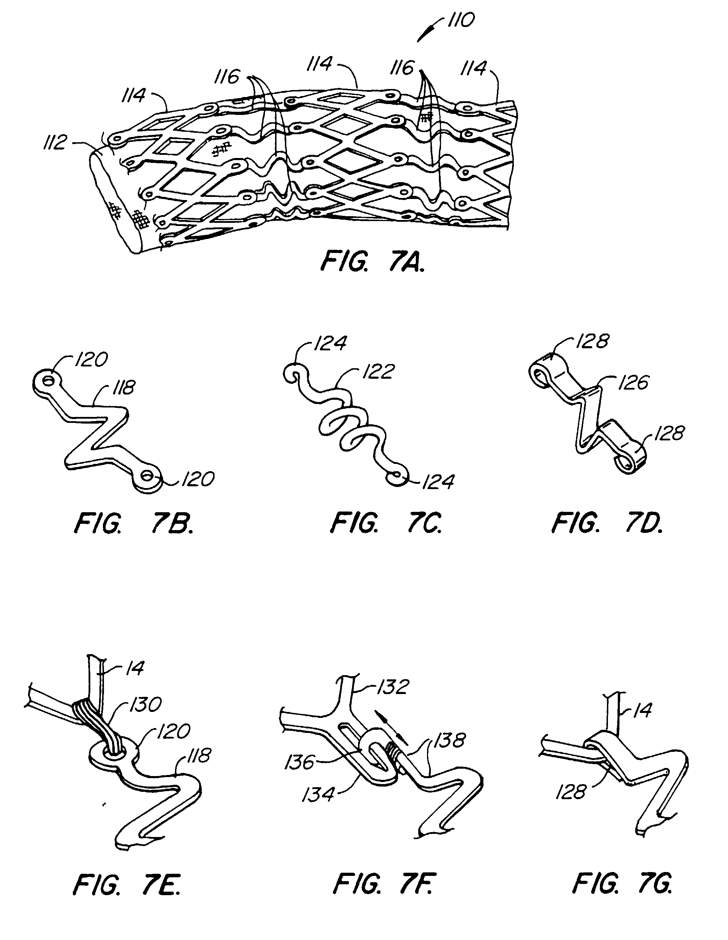

A particularly advantageous flexible prosthetic structure 110 will be described with reference Figs. 7A-G. Flexible structure 110 comprises a radially expandable liner 112 supported by a plurality of ring frames 114. A series of connector elements 116 extend between adjacent ring frames 114. Optionally, connector elements 116 may also be used to support the liner 112. Advantageously, the connector elements and ring frames may be independently optimized to tailor the mechanical properties of the prosthesis structure, particularly for use as a flexible trunk or branch position in the branching prosthesis of Fig. 3A. Alternatively, flexible prosthetic structure 110 may find use as a stent, or as a cylindrical stent-graft.

Preferably, the ring frames comprise resilient self-expanding structures, ideally comprising a super-elastic shape memory alloy such as Nitinol™. Connectors 116 preferably comprise a malleable material, ideally including martensitic Nitinol™, stainless steel, cobalt-nickel alloy, titanium, or tantalum. Clearly, the connector elements can provide additional column strength to the prosthetic structure, as well as providing support to the liner between the ring frames. Advantageously, such malleable connectors may also provide a structure which will expand resiliently when deployed *in situ*, and which will conform plastically to an axially ortuous body lumen, such as the blood vessels of the vascular system.

Preferably, connector elements 116 comprise serpentine elements which extend axially between adjacent frame loops. Careful selection of the serpentine shape allows tailoring of the bending properties of the prosthesis. Such serpentine connector elements located at the outer portion of an axial bend in the prosthesis will be straightened, while those at the inner portion will decrease in length, optionally maintaining the axial length of the prosthesis at a relatively constant amount. Alternatively, the connector elements may rely primarily (or solely) on either elongation or compression alone, thereby inducing changes in the length of the prosthesis when bent.

Figs. 7B-D illustrate alternative connector element structures. A flat connector element 118 may be cut from a flat sheet of the desired malleable material, and optionally includes ends 120 having passages cut therethrough to facilitate attachment of the connector element to the resilient frame structure. Such a flat structure has the advantage of not decreasing the internal prosthetic lumen cross-section within a narrow body lumen, and the flat serpentine shapes may be cut from sheet stock using known laser cutting, lithography techniques, or the like.

Alternatively, a wire connector element 122 having bent loop ends 124 may be formed as a helical coil. In a still further alternative, a bent connector element 126 may be formed from a straight strip of malleable material, as shown in Fig. 7D, and may also include folded ends 128. Clearly, a wide variety of alternative metallic or polymer connector structures may be suitable. Generally, it will be preferable to make use of materials which are both malleable and biocompatible, as described above.

A variety of alternative attachment mechanisms for coupling the frame structure to the connector elements are shown in Figs. 7E-G, and also in Fig. 7A. Generally, the connector elements may be attached to the frame loops by welding, soldering, adhesive bonding, polymer rivets, suturing, or the like. In some embodiments, it may be possible to utilize members which extend from a resilient frame, and which have been formed to the desired shape and heat treated or otherwise processed to produce the desired malleable properties. In some embodiments, the mechanism used to attach the resilient frame to the connector elements will also attach the liner to the frame, for example, stitching which extends through passages in both the connector elements and the frame, and then through a woven textile liner.

It may be desirable to allow some longitudinal motion between the connector elements and their associated frames without deforming the connector elements. An oversized suture loop 130 between a ring frame 14 and passage 120 of flat connector element 118 provides a limited amount of axial motion. Similarly, an axial slot 134 in a slotted frame 132 provides a precisely controlled amount of axial motion of a loop 136 on a wire connector element 138. Note that loop 136 may further be reinforced by suture, wire, adhesive, or the like. Alternatively, the end of the connector element may be folded over a ring frame 14, and optionally adhesively bonded in place, to provide a positive connection.

Preferably, connectors 116 compress or elongate plastically under forces typical of those imposed on the prosthesis during deployment. As these forces are typically higher than normal physiological forces, the connector elements may advantageously be constructed to avoid deformation from these normal blood and tissue *in vivo* forces, particularly where a limited amount of axial motion is allowed between connector elements and the ring frames. Therefore, the prosthesis structure can plastically deform during deployment to conform the axis of the prosthesis with the surrounding body lumen, but will thereafter avoid imposing resilient straightening forces against the body lumen.

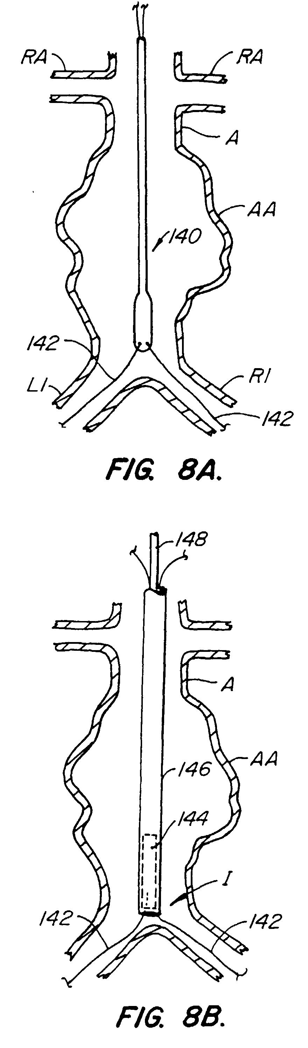

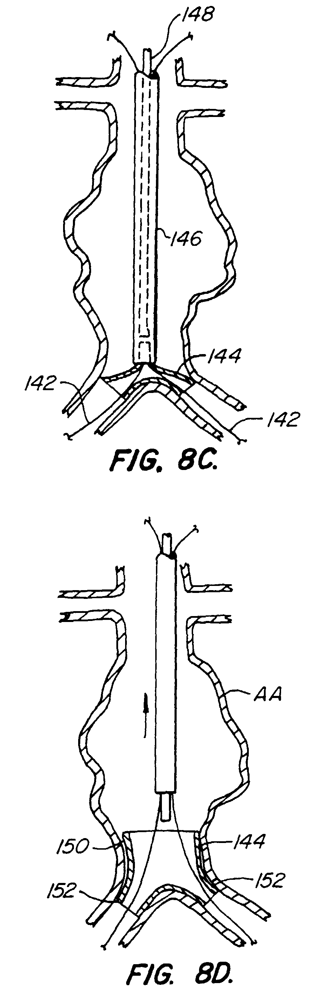

A method for assembling *in situ* an endoluminal prosthesis by first positioning and deploying a hub module will be described with reference to Figs. 8A-E. A branch access catheter 140 is used to insert guidewires 142 down the aorta A and into the left iliac LI and right iliac RI. The branch access catheter 140 preferably comprises a deflecting tip branch access catheter as taught by U.S. Patent No. 4,774,949.

A resilient hub module 144 is advanced over both guidewires 142 while compressed within delivery sheath 146. Hub module 144 preferably comprise an elastic sponge-like microporous silicone, silicone foam, low durometer silicone, polyurethane foam or the like, as more fully described in co-pending U.S. Patent Application Serial No. 08/525,989, filed September 8, 1995. Hub module 144, which may be stented or unstented, is deployed over guidewires 142 at the luminal intersection I of the aorta A and left and right iliaci LI, RI, optionally extending along the iliaci beyond the aortic aneurysm AA. Ideally, hub module 144 is deployed by a combination of distally advancing pusher shaft 148 and proximally withdrawing catheter sheath 146 so that a trunk portion 150 of the hub module remains within the aorta, while branch portions 152 extend into each of the iliaci. The hub module wall material will preferably be at least in part self-supporting, but may be reinforced adjacent the trunk or branch ports for sealing and to provide sufficient hoop strength to allow prosthetic modules to sealingly engage the hub from within.

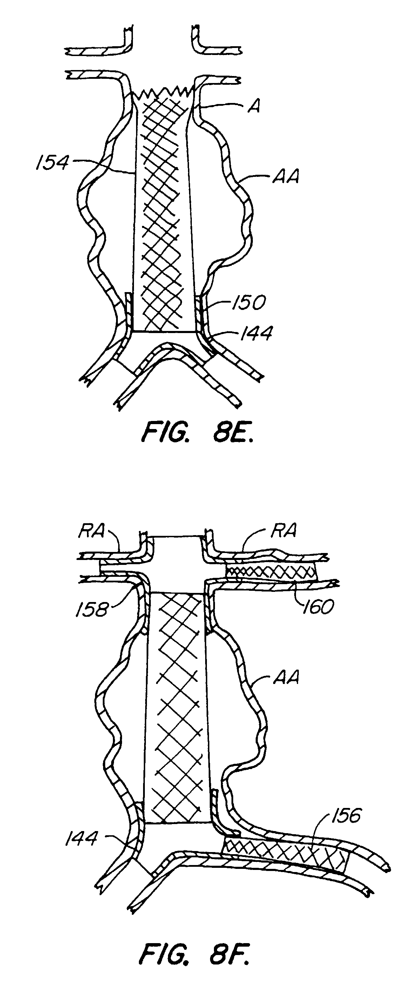

In some embodiments, it may be possible to completely seal off aortic aneurysm AA by positioning a trunk module 154 within trunk port 150 and expanding the trunk module to sealingly engage the hub module and the healthy aorta upstream of the aneurysm, as illustrated in Fig. 8E. In other cases, it may be necessary to extend one or more branch modules 156 along one or both iliac arteries to fully bypass the aneurysm, as illustrated in Fig. 8F. A four branch hub module 158, similar in structure to hub module 144, may find use in sealing off the upper end of an aneurysm which extends to or along the renal arteries, optionally making use of a renal branch module 160 similar to branch module 156 described above. Optionally, one or more hubs may be securely attached to (and deployed with) a trunk stent-graft.

Although the exemplary microporous silicone can adapt to a range of luminal intersection geometries, it may be advantageous to provide a variety of hub modules having differing angles to accommodate a wider variety of vascular geometries, allowing selection of a suitable hub for each patient. In extreme cases, it may even be preferable to custom mold a hub module for a specific patient's vasculature, preferably based on information provided by fluoroscopy, ultrasound, or some other imaging modality.

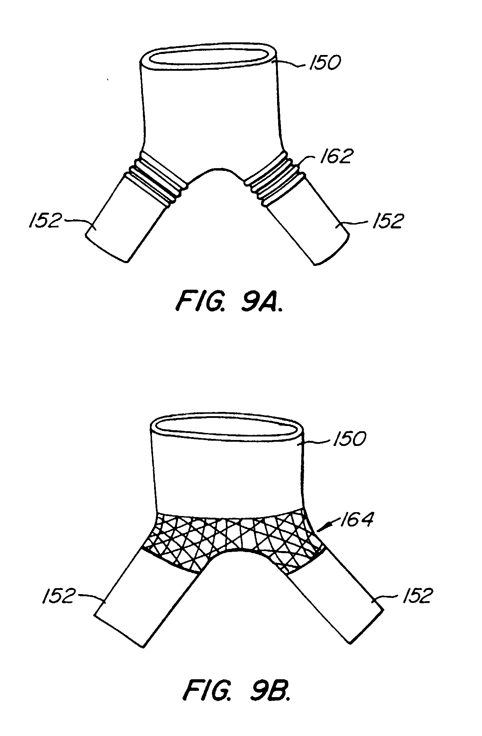

To increase the ability of the hub module to conform to a variety of vascular geometries, it may be advantageous to include corrugated portions 162 or braided portions 164 between the trunk port 150 and the branch ports 152 as illustrated in Figs. 9A-D. Such corrugated structures accommodate compression along the inside of a tight bend radius without kinking, while braided structures are inherently kink resistant when bent. Similar enhanced flexibility portions may be used at the junctions of a trifurcation to increase the conformability of an aortal renal hub module, similar to renal hub module 158 shown in Fig. 8F. Advantageously, first deploying the hub module adjacent the intersection of the aorta and iliac arteries allows the trunk module to be supported at least in part from the luminal intersection, particularly during deployment.

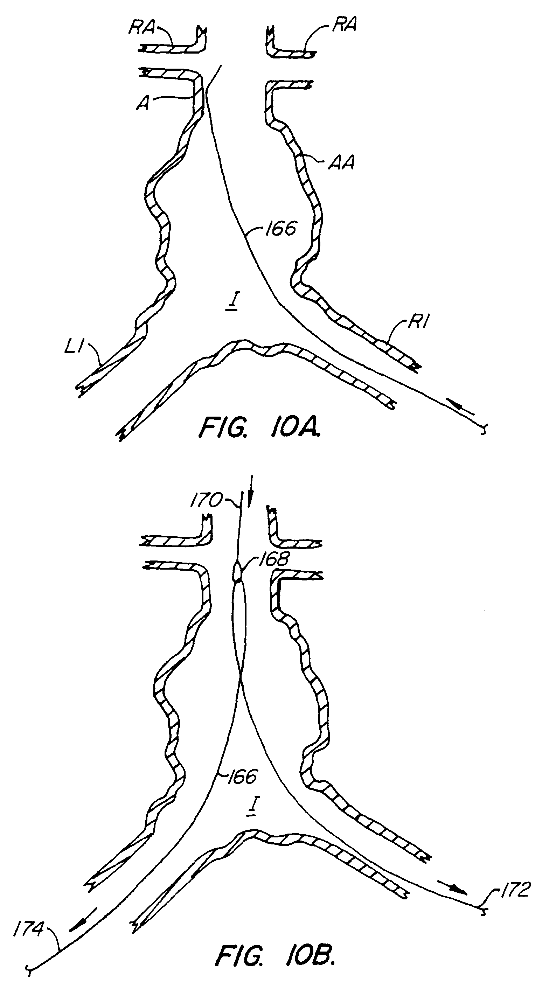

A method for precisely positioning an endoluminal prosthesis using guidewires which pass through the luminal intersection will be described with reference Figs. 10A-C. In the exemplary method, a guidewire 166 is introduced from an inferior position and advanced along the right iliac, RI beyond the luminal intersection I, through the aorta A to the subclavian or carotid artery, where the guidewire is extended out of the patient body. Guidewire 166 can then be threaded through a loop 168 in a second guidewire 170, and the distal end of guidewire 166 be again maneuvered back through the aorta, beyond the luminal intersection I, along one of the iliac arteries, and again extended out of the patient body.

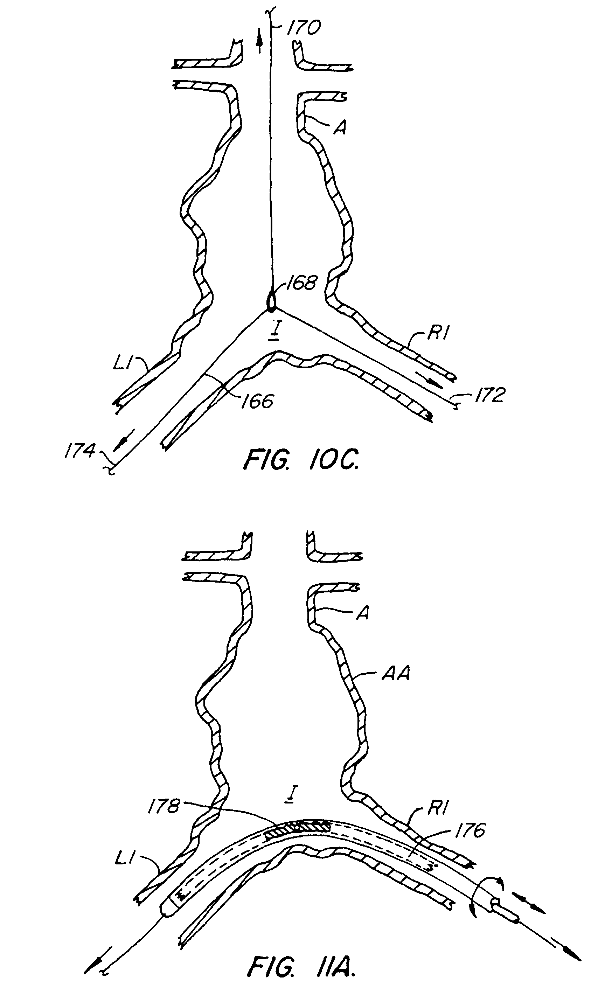

Advantageously, a proximal end 172 and distal end 174 of guidewire 166 may be selectively tensioned to advance second guidewire 170 down the aorta to the luminal intersection I. Optionally, guidewire 166 may be fed inward and outward through the same iliac to allow loop 168 to be positioned relative to the aortic and one of the two iliac arteries. Alternatively, as shown in Fig. 10C, guidewire 166 may be fed inward through, and outward from, alternative iliac arteries. In either case, tensioning the proximal and distal ends of guidewire 166 and the proximal end of second guidewire 170 precisely positions hoop 168 relative to the luminal intersection I. Hence, this method provides multiple points of control and access to fine tune endoluminal prosthesis placement, and allows prosthetic modules to be advanced along either end of guidewire 166 or along second guidewire 170 to the precisely positioned loop 168.

As described above, many bifurcated stent-graft systems depend on attachment to a narrow healthy or less diseased zone between the renal arteries and the upstream end of the aneurysm. The length and diameter of this healthy zone can be very difficult to predict, making secure attachment and sealing of the endoluminal prosthesis problematic. As there may be little or no healthy aorta remaining between the aneurysm and the renal arteries to anchor a branching endoluminal prosthesis, it would be advantageous to find alternative support mechanism for branching endoluminal prostheses.

As was also described above, the iliac arteries may define substantial angles relative to the aorta, particularly on patients having abdominal aortic aneurysms. This often complicates the positioning of a tightly compressed (and therefore relatively stiff) endoluminal prosthesis across the lumenal intersection from the aorta to the iliac arteries.

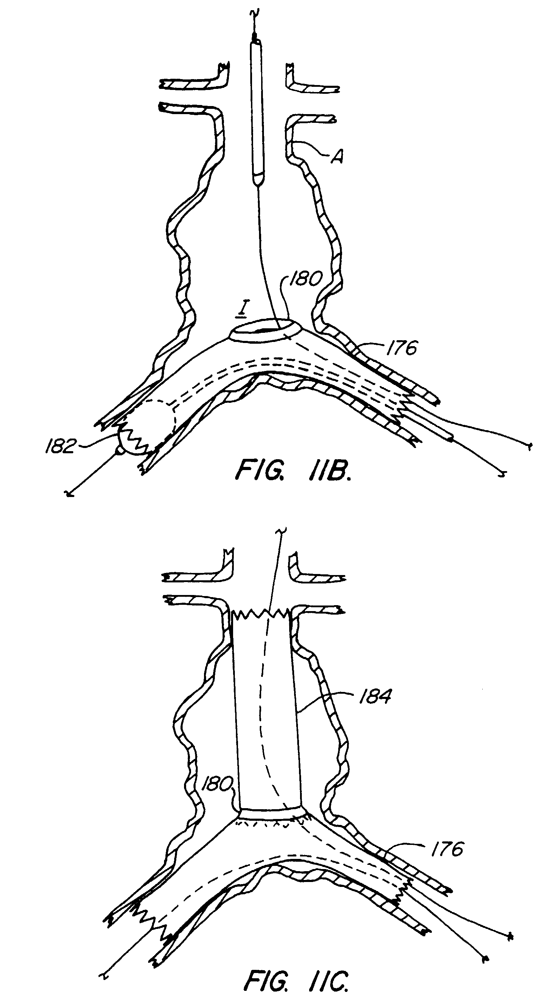

For these reasons, it may be advantageous to instead position and deploy a branch module 176 extending across the luminal intersection I from the right iliac to the left iliac, as illustrated in Fig. 11A. Branch module 176 will generally include a trunk port 180 which is preferably oriented along the aorta, as shown in Fig. 11B. Such orientating of prosthetic modules is aided by a radiographic marker 178 which provide a visual representation of the expanded module under imaging. Optionally, a balloon catheter 182 may be used to hold branch module 176 in position during deployment of a trunk module 184 into sealing engagement with trunk port 180.

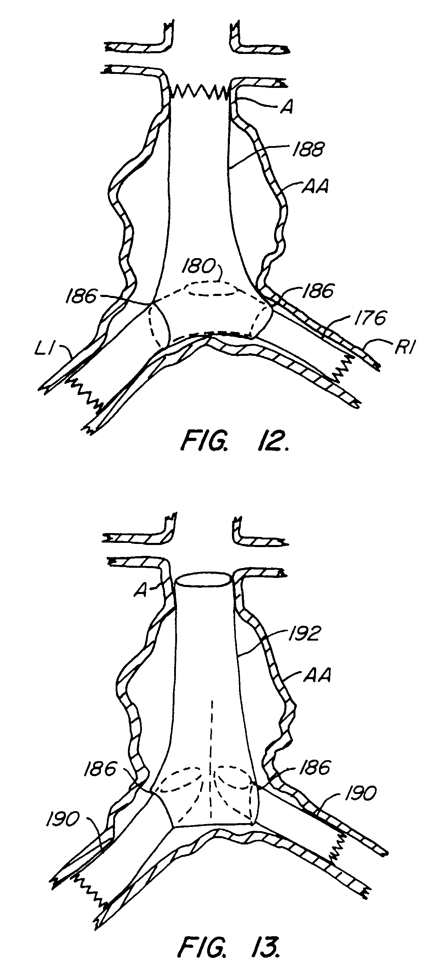

Referring now to Fig. 12, branch module 176 may alternatively be deployed through branch ports 186 of a previously deployed primary trunk module 188. Flow for the two iliaci thus enters the branch module within the lumen of primary trunk module 188 through trunk port 180. A somewhat similar arrangement, which makes use of independent branch modules 190 that sealingly engage an alternative primary trunk module 192 at branch ports 186, is illustrated in Fig. 13.

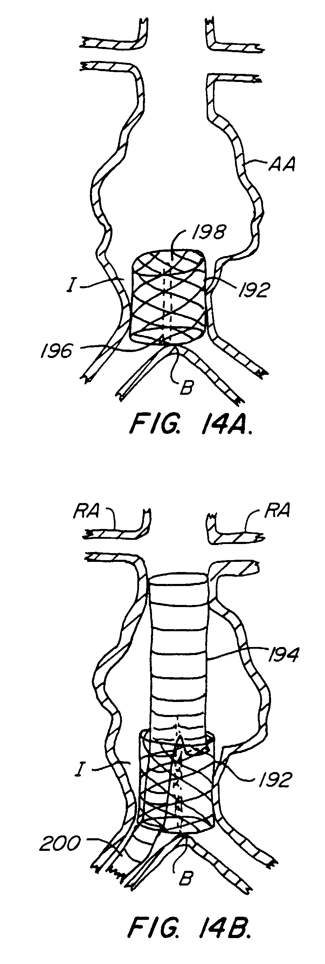

It would be advantageous to provide still further alternative methods for supporting the endoluminal prosthesis assembly, rather than relying substantially on the aorta below the renals. As illustrated in Figs. 14A-B, a spacer module 192 may first be deployed adjacent the luminal intersection I, preferably with a lower surface 196 in contact with the bifurcation B of the body lumen system. Spacer module 192 is selected so that an upper surface 198 is at the proper distance from the lower surface 196 so that a bifurcated trunk module 194 resting on upper surface 198 is correctly positioned just downstream of the renal arteries. Branch modules may then be positioned through the spacer module and into the branch ports of the bifurcated trunk module to complete the bifurcated prosthesis assembly. Clearly, one or more of the branch portions may optionally be formed integrally with the trunk portion, within the scope of the present invention.

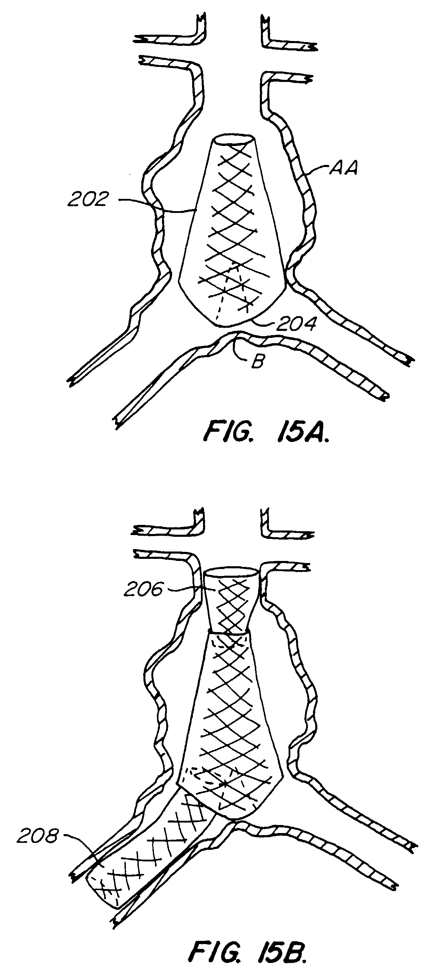

A still further alternative modular prosthetic assembly will be described with reference to Figs. 15A-B. Tapered primary module 202 includes a wide branch end 204 which is optionally deployed within the luminal intersection so as to be supported by the body lumen bifurcation B. Advantageously, the wide branch end 204 facilitates engaging branch modules 208 from widely divergent iliac arteries, and may also help support a trunk lumen sealing module 206 from the bifurcation of the body lumen, as shown in Fig. 15B.

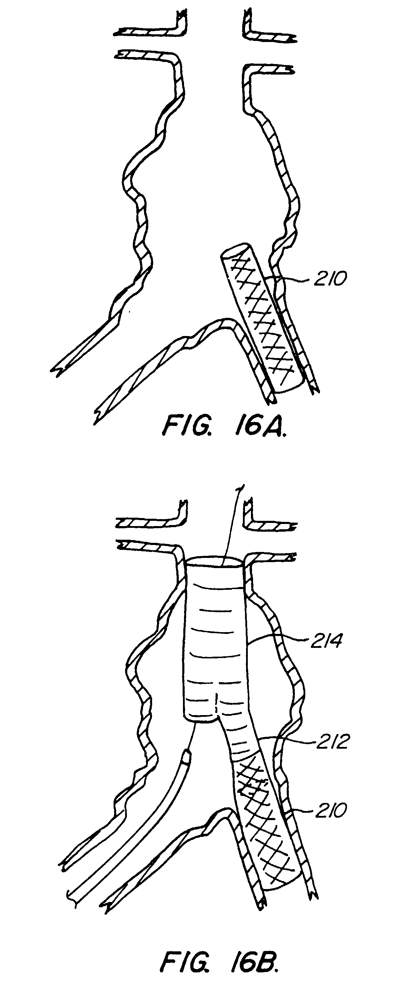

It also provides supporting *in situ* endoluminal prosthesis assembly from within the iliac, as illustrated in Figs. 16A-B. In this embodiment, a branch prosthetic module 210 is first deployed within a relatively healthy renal artery. One branch port 212 of a bifurcated prosthetic module 214 is then positioned and expanded within branch module 210. Optionally, a second branch module is then positioned within an alternate branch port of the bifurcated modules 214, completing the *in situ* assembly of the bifurcated prosthesis system.

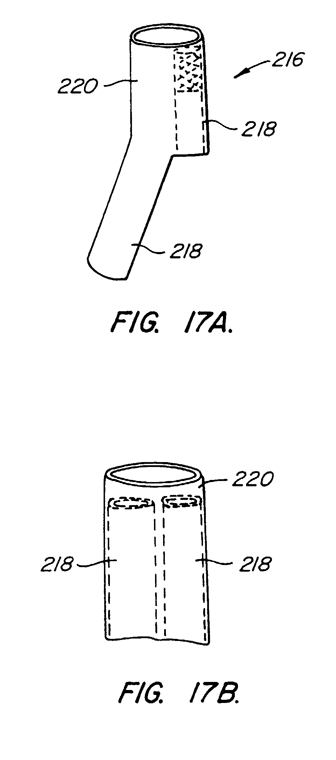

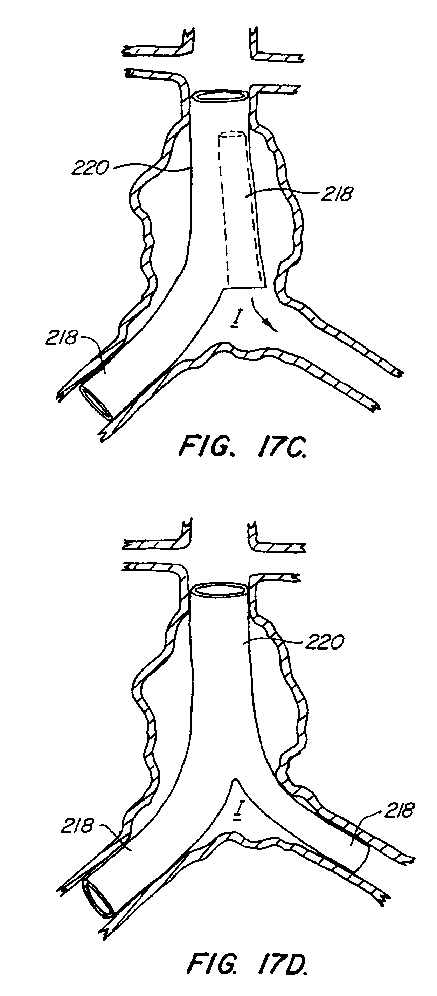

It would be desirable to reduce the number of prosthetic module deployment steps required to deploy an endovascular bifurcated prosthesis system. Toward that end, as shown in Figs. 17A-D, an extendable leg bifurcated prosthesis 216 may have one or more leg portions 218 disposed within the trunk portion 220 when the prosthesis is radially compressed for positioning and deployment. Optionally, the leg may be averted within the trunk portion, the leg preferably comprising a self-supporting or composite material. Alternatively, the leg may slidingly engage the trunk portion and telescope out into position. In either case, disposing the leg within the trunk portion greatly facilitates positioning the prosthesis across the luminal intersection I.

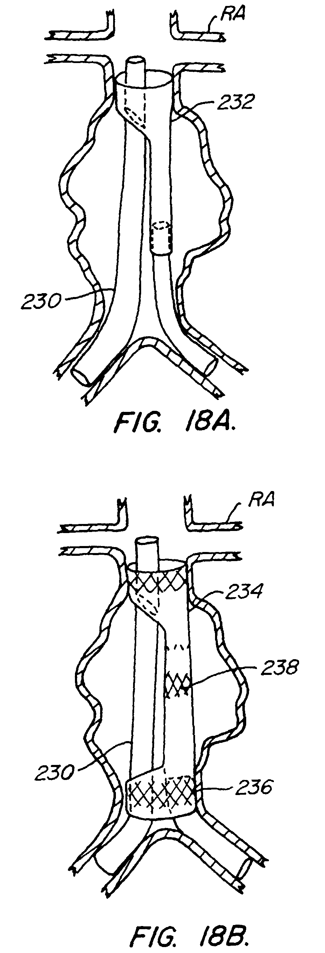

The location and extent of aneurysms along the renal arteries varies considerably between patients, and may at times be difficult to accurately measure. It would therefore be advantageous to provide modular structures adaptable to a wide range of iliac leg positions. The prosthetic assemblies of Figs. 18A-B achieve such iliac leg placement flexibility by extending a relatively rigid iliac module through bifurcation modules 232, 234, optionally even allowing iliac module 230 to extend in cantilever beyond renal arteries RA. Additionally, by minimizing the length of the trunk lumen portion of the prosthesis, the mass of each module is minimized, facilitating intravascular maneuvering.

To provide some mutual support between the parallel iliac portions, bifurcation module 234 includes a lower support portion 236 having the two-lobed cross-section which is described in co-pending Patent Application Serial No. 08/538,706 (1995). The relatively narrow mid-section 238 allows axial bending of the assembled prosthesis through the aneurysm to adapt to physiological movement.

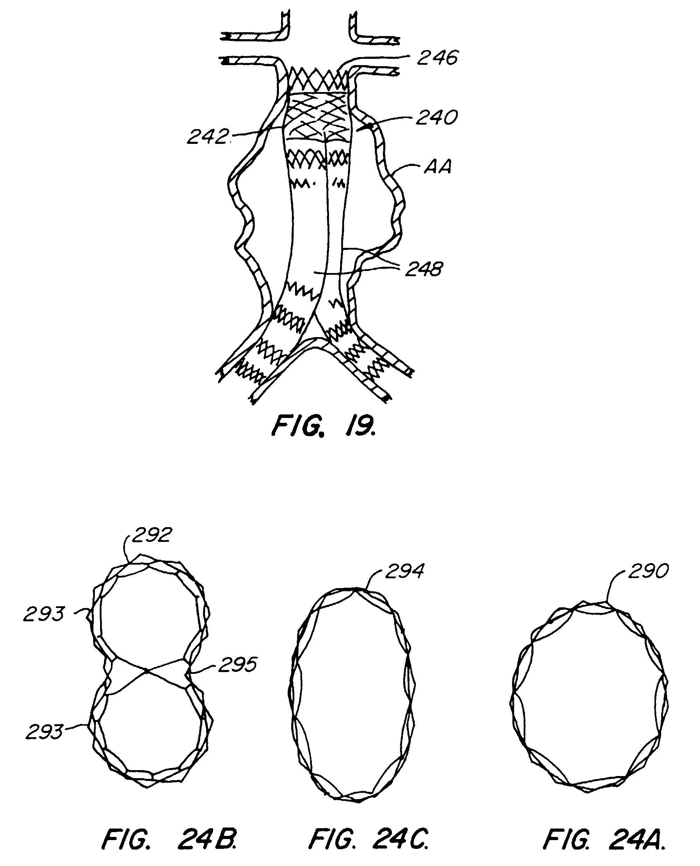

Referring finally to Fig. 19, a short trunk branching prosthesis includes a lumen separation portion 242 which is adjacent to a trunk sealing cuff 246, here shown as a single independent ring frame which is crown stitched to the liner. Advantageously, the branch portions 248 will tend to have good axial flexibility due to their significantly smaller diameter than the trunk. Hence, the branch portions may be supported by independent ring-frames.

The modular prostheses, which will generally be formed as stent-grafts, provide significant advantages for the endovascular treatment of abdominal aortic aneurysms. Along with the ability to adapt to highly varying lumenal geometries, modular prosthetic systems can be used to avoid the leakage of blood around the proximal or distal end of the prostheses, can help to ensure collateral circulation is maintained, can avoid or accommodate device migration, minimize thrombosis regression, and are particularly advantageous at adapting to disease progression after the prostheses are initially deployed.

The geometric adaptability of modular prosthetic systems allow therapies to be adapted to accommodate various access approaches, angulation of the lumenal intersections, lumenal diameters, different locations and extent of healthy vessel walls available for fixation, and varying requirements for circumferential conformability. These various requirements can be accommodated by several individual prosthetic modules, each of which presents a small insertion profile to facilitate either femoral or sub-clavian introduction and positioning. Branches can be specifically designed to accommodate wide areas of angulation, yet be relatively resistant to compression and kinking. The assembly procedure is preferably performed under real-time fluoroscopy, with the surgeon selecting and assembling the prosthetic modules so as to best suit the anatomic pathology as the assembly procedure progresses.

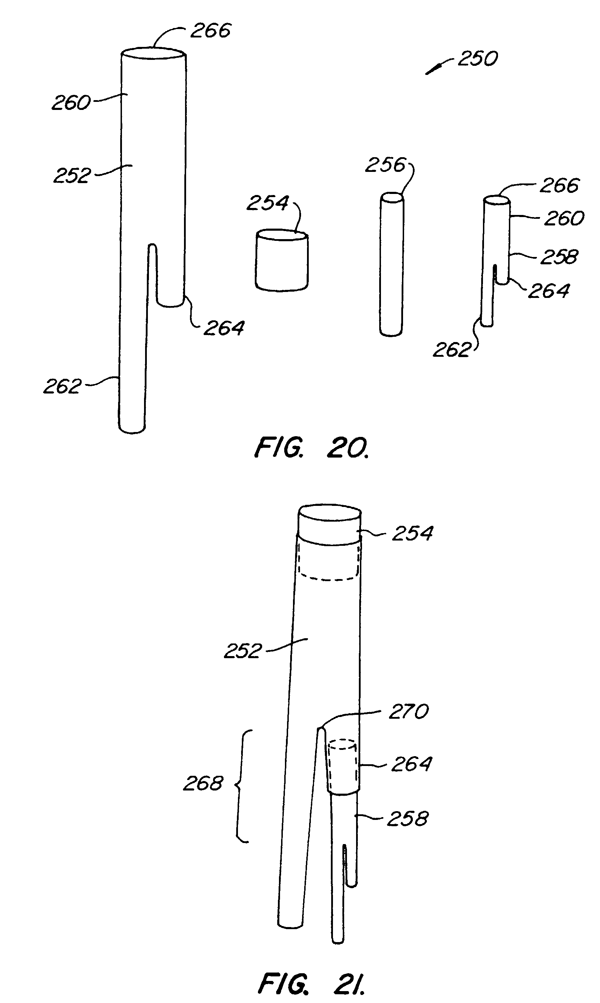

The components of an exemplary bifurcated endoluminal stent-graft system 250 for treatment of an aorto-iliac aneurysm are schematically illustrated in Fig. 20. Major components include a main bifurcation module 252, an upstream sealing cuff 254, a straight extension module 256 and a bifurcated extension module 258. The main bifurcation module 252 and the bifurcated extension module 258 each include a trunk portion 260 and first and second branch portions 262, 264. Trunk portion 260 includes a trunk lumen 266, while branch portions 262, 264 each have a branch port in fluid communication with the trunk lumen. When the prosthetic system is assembled as shown in Fig. 21, it provides a sealed prosthetic lumen system having branches which are separated by a branch separation distance 268.

It is a particular advantage of the bifurcated prosthetic system 250 that the branch separation distance 268 can be varied by varying the overlap between adjacent prosthetic modules. To enhance the linear and angular variability of the prosthetic system, branch portion 264 (to which the extension will be sealed) extends a substantial distance beyond the main bifurcation 270, and second branch portion 264 may also be axially flexible independently of the first branch portion 262.

The axial length of main bifurcation module 252 from the end of trunk portion 260 to the opposed end of first branch portion 262 will generally be between about 9 and 20 cm, preferably being between about 11 and 16 cm. The bifurcated extension module will typically be between about 5 and 15 cm in axial length between the opposed ends of its trunk and branch portions, ideally being between about 6 and 10 cm. The axial length of sealing cuff 254 will generally be between about 2 and 8 cm, ideally being between about 3 and 5 cm in length.

The axial dimensions of the assembled prosthesis can be varied by varying the axial overlap between adjacent modules within some predetermined overlap range. Some minimum overlap, generally between about .7 cm and 1 cm, helps to ensure adequate module/module fixation and sealing. Maximum overlap limitations may improve lumenal continuity and avoid stagnation zones (and the resulting thrombus). For example, it will often be desirable to avoid insertion of an extension module within second branch portion 264 beyond the lumenal bifurcation 270 of main bifurcated module 252, so that the extension does not fold over and occlude lumenal flow to one or both branches, and so that thrombus does not accumulate between the outer surface of the extension module and the nearby main bifurcated module trunk lumen. Therefore, it will often be helpful to provide a substantial distance between bifurcation 270 and the end of second branch 264 to enhance axial variability. Generally, the predetermined overlap variability range will be at least 2.5 cm, ideally being at least 3.5 cm.

Providing acceptable overlap variability ranges may be complicated by other design considerations. For example, as described above regarding Fig. 19, minimizing the length of the large diameter trunk and maximizing the length of the smaller, more flexible branch portions may facilitate some deployments. Thus, the length between the beginning of the prosthetic trunk lumen and the opposed end of the second branch 264 might be anywhere from about 2 to 10 cm. While the longer trunk lengths can easily accommodate the desired minimum overlap variability ranges, shorter main bifurcation modules could potentially limit overlap. Hence, during some prosthetic deployment procedures, it may be preferable to insert the extension module beyond the prosthetic lumenal bifurcation, or even beyond the trunk lumen, to avoid occluding a downstream branch. For example, deployment of a selected straight extension module 256 within main bifurcation module 252 to isolate an abdominal aneurysm may distort the diseased vascular geometry to the point that the extension module extends downstream beyond the hypogastric artery unless the extension module is inserted beyond bifurcation 270. In such circumstances, it may be preferable to maximize overlap variability by inserting the module as far as required to leave the hypogastric branch open. Therefore, when intended for use with relatively short bifurcation modules, extension module structures may optionally have sufficient column strength to be extended some distance in cantilever beyond the bifurcation septum. The predetermined adjustability range will then include that cantilever range.

The total length and diameter of the assembled prosthetic system can also be varied by selection of prosthetic modules. The basis for this selection can be imaging and diagnostic tests performed prior to the deployment procedure, optionally employing Computer Tomography (CT), X-ray/fluoroscopy, ultrasound/intravascular ultrasound (IVUS), or the like. Advantageously, module selection may also reflect the physiology as impacted by the previously deployed modules, and as viewed under the fluoroscopy or other imaging modality used to direct deployment. For abdominal aneurysm therapy, the distance between the branches of the multiple branch prosthetic lumen system may be between about 10 and 30 cm to provide sealed prosthetic lumens from below the renals to a hypogastric artery.

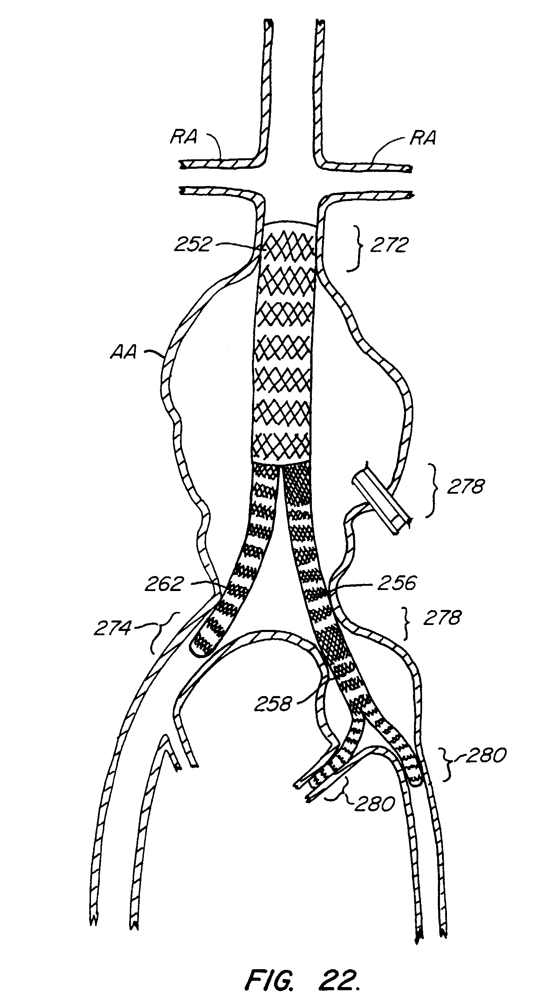

The use of the bifurcated prosthetic system of Fig. 20 can be understood with reference to Fig. 22. The main bifurcation module may be introduced either from upstream (a sub-clavian, antigrade, or superior approach) or from downstream (a femoral, retrograde, or downstream approach). The longer branch portion 262 of main bifurcation module 252 will generally extend downstream beyond the aneurysm AA, while straight and/or bifurcated extensions are added to the shorter leg 264 so that the bifurcated prosthetic system extends downstream beyond the aneurysm as shown. The number, length, and type of extension modules are selected to isolate the aneurysm, and so that the prosthetic lumen can be sealed against healthy lumenal walls.

The upstream sealing module 254 optionally allows upstream length adjustment, generally ensuring a seal between the prosthetic lumen and body lumen system by having a locally increased expansive force, or the like. Such sealing cuffs allow the surgeon to make appropriate adjustments by overlapping the sealing module with the trunk of the main bifurcation module until the correct main lumen length is provided.

Adaptability of the fixation location 272 is particularly advantageous. During introduction, the stent-graft delivery system can be moved axially within a reasonable range prior to the initial of stent-graft deployment. When the first stent ring of the stent-graft has been deployed, the main module becomes relatively fixed. Advantageously, where independent stent rings support the liner, the unexpended portion of main bifurcated module 252 can still be moved a short distance if needed to provide the final position of the module.

When using an inferior or femoral approach, initial deployment of the stent-graft usually begins with the main bifurcated portion slightly upstream its final target position. The prosthetic modules will generally be drawn proximally during deployment so as to avoid injury to the vascular wall which might result from distal movement of runners 42 or some other portion of the deployment system (see Fig. 2). At completion of the deployment of the main bifurcated module 252, the trunk lumen extends to just below the renal arteries.

As described above, the deployment may alternatively make use of an anti-grade sub-clavian artery approach, rather than a retrograde femoral artery approach. The stent-graft will generally be enclosed within the delivery system in an orientation specific to the introduction pathway. Marking of the orientation of a compressed stent-graft on the surrounding delivery system, pre-loaded cartridge, or the like helps to ensure that the stent-graft will be properly oriented upon its deployment.

Dimensional adaptability preferably provides both length and diameter variability, so that the prosthetic system can be appropriately matched to the particular body lumen geometry. Diameter conformability allows the prosthesis to adapt to irregular cross-sections to provide sealing, while length conformability allows the prosthesis to flex without kinking to accommodate physiological movement between a fixed upstream attachment region 272 and a fixed downstream attachment region 274. In some embodiments, it may be possible to leave unfixed downstream attachment regions to increase axial conformability, but this may have disadvantages with regarding to peripheral sealing.

Referring still to Fig. 22, the modular prosthetic assembly allows axial length to be adjusted by moving the overlapping portions 278 of the stent-graft modules relatively to one another during or after the deployment process. The left portion of the prosthetic system of Fig. 22 provides somewhat limited linear dimensional variability, and therefore relies primarily on the conformability of the stent-graft structure to accommodate physiological changes between the upstream fixed location region 272 and the downstream fixed location region 274. The right side of the stent-graft system shown in Fig. 22, which would be the contralateral side relative to deployment of the main prosthetic module 252, terminates in an overlap region 278 at the downstream end of the second branch.

Once the main bifurcation module of the stent-graft is deployed, the dimensions of the body lumen surrounding this contralateral side may change significantly due to straightening of the angle of the aneurysmal neck below the arteries, and the like. It is in this region where adjustable overlap regions 278 will allow the physician to move the stent-graft straight extension module 256 and bifurcated extension modules 258 up or down for axial conformability.

Cross-sectional dimensional variability is important to effectively achieve an adequate seal and isolate the weakened aneurysm from the bloodflow. Such variability is particularly important in atherosclerotic vessels with prominent calcific plaque. To help ensure an adequate seal, the stent-graft will preferably be slightly oversized. In some embodiments, the artery can be molded to the cross section of the graft, while in other embodiments the graft can conform with the irregular arterial cross-section. Optionally, the upstream and downstream seals can be enhanced by use of a conformable stent-graft, in combination with balloon expansion of the end portions, to provide effective sealing of the ends and prevent leakage into the aneurysm.

Referring once again to Fig. 22, seals are established around each end of the prosthetic lumen to ensure that the aortic aneurysm is isolated. Main bifurcation module 252 is sealed at a fixed attachment point 272 and at a fixed downstream attachment region 274. To provide blood flow to both the hypogastric and femoral arteries, and to completely isolate the aneurysm AA, bifurcated extension module 258 is also sealed at each of its downstream branch regions 280. Linear dimensional adaptability is provided by two overlap regions 278, where the straight extension module 256 overlaps the main bifurcation module 252, and where the branching extension module 258 overlaps the straight extension module 256.

Advantageously, the branching extension module 258 can be deployed in the same procedure as the main bifurcation module to provide a therapy of an aneurysm which extends substantially along an iliac artery, or the branching extension module may be added in a later procedure to accommodate disease progression long after the original bifurcated system was deployed. Similarly, a branching hub module may be introduced at the renal arteries RA (as described regarding Fig. 8F) in either the initial deployment procedure, or in a follow-up procedure.

The components of the modular system are preferably designed to maximize versatility and to accommodate a wide variety of lumenal geometries at the initial deployment procedure for exclusion of the abdominal aortic aneurysm. Additionally, the components should maintain structural integrity for long term treatment of the disease. The stent-graft components preferably comprise a shape memory alloy such as Nitinol™, which serves as an exo-skeleton to provide column strength and flexibility, and to resist compression and kinking. While such a stent structure is radiopaque, the modules preferably include highly visible marker elements at selected point to facilitate device visualization during implantation.

In some embodiments, the graft material is a thin walled, un-crimped, knitted polyester, such as Dacron™, which contributes to the low profile of the introduction and delivery system and facilitates a healing response via tissue in-growth. The modules are preferably collapsible to a very small cross-section.

The delivery system will preferably be compatible with present and evolving catheter technologies, including intra-vascular ultrasound (IVUS). Precise deployment of the prosthetic system in complex or difficult cases is facilitated by accessing the target side through both the retro-grade femoral and anti-grade sub-clavian approaches. In some embodiments, the upstream and downstream ends of the device possess diameter and length variability to accommodate individual anatomical differences, while the region of the primary bifurcation ensures devices stability through relative rigidity. A self expanding shape memory alloy stent and polyester graft combination is particularly advantageous for sealing at the upstream and downstream attachment regions, while maintaining high column strength and a stable structure.

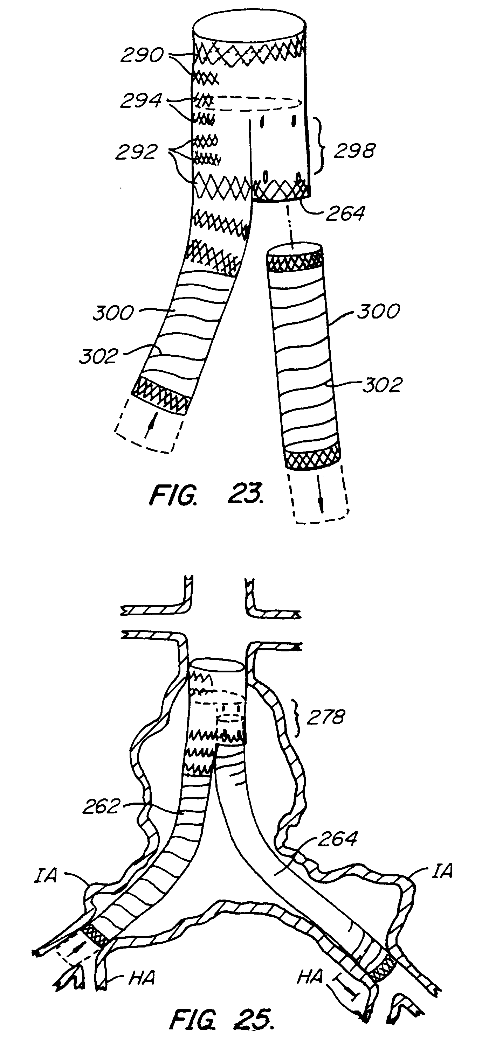

Referring now to Fig. 23, a preferred bifurcated modular structure makes use of a series of specialized ring frames to support a liner, thereby providing lumenal patency and axial flexibility in a single integral structure.

Trunk ring frames 290 are biased to expand to a substantially circular cross-section for anchoring and sealing, as shown in Fig. 24A. Peanut ring frames 292 are biased to expand to a two lobed cross-section in which lobes 293 are separated by an isthmus 295, as shown in Fig. 24B, and as more fully described in co-pending U.S. Patent Application No. 60/008,254, 1995. Each of the lobes supports an associated branch lumen downstream of bifurcation 270, while the isthmus helps maintain the intended flow balance between the branches when an extension module is introduced into one branch. Transition ring frames 294 will provide a smooth transition between the trunk rings 290 and peanut rings 292, and will typically be biased to expand to an elliptical or oval cross-section as shown in Fig. 24C. The use of two trunk rings, two transition rings, and three peanut rings is particularly preferred, although a wide variety of alternative combinations are also possible. The integral leg will optionally be supported by ring frames similar to trunk frames 292 but smaller in cross-section.

Independent ring frames enhance axial flexibility of the bifurcated prosthetic module both after its deployment and while the module is compressed within the delivery system. Trunk rings 290 will be attached to the liner so that the trunk portion has sufficient axial flexibility to accommodate the aortic dive often found just downstream of the renals, optionally being sewn outside the liner with a stitching pattern that allows the rings to move relative to each other. The peanut frames may optionally be attached over the outer surface of the liner with a stitch which locally limits the module's axial flexibility, or may instead primarily rely on the separate lobes of the independent peanut frames to maintain lumenal patency. The lumenal bifurcation 270 is separated from the bottom of the second branch 264 by sufficient distance to provide at least 2.5 cm of overlap adjustability, the overlap adjust range here being indicated by a gate 300 formed with discrete radiopaque markers.

The advantages of an optional axially conformable branch structure 300 can be understood with reference to Figs. 24 and 25. The ends of the conformable branches are anchored and sealed with ring frames as described above, the rings typically forming linked diamond shape D-elements, zig-zag bends, coils, or the like, and will provide a relatively high radial expansion force.

The liner between the ends will allow axial elongation and/or compression of the branch, but will not occlude flow, the liner typically being corrugated, redundantly bunched or averted, an axially oriented resilient material, or the like. A coil 302 also accepts changes in axial length, and keeps the lumen open. Hence, in addition to the variability provided by the adjustable overlap region 278, the length of one or both branches can be adjusted, even after the position of one end has been fixed. This is particularly advantageous for sealing in precise axial locations, such as downstream of an iliac aneurysm IA and upstream of a hypogastric artery HA, as shown. Adjustment of the first branch 262, which is integral with the bifurcation module, or second branch 262 after the opposite end has engaged the bifurcated module may be facilitated by runners 42 within the delivery system (See Fig. 2).

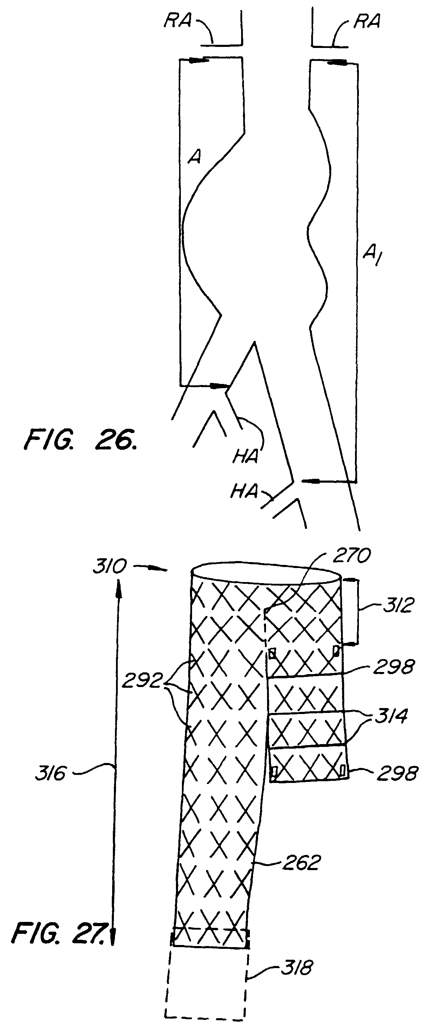

As described above, the distance between the renal arteries and the hypogastric arteries can vary considerably. Referring now to Fig. 26, these distances A, A' may even vary substantially within a single patient. The measurement of these distances is very important to a proper prosthetic therapy. If the selected device is too long, the hypogastric branch may be occluded. If the device is too short, the aneurysm may not be isolated. While it is possible to provide a large number of selectable module lengths and rely on the accuracy of lumenal measurements, the inventory costs of such a system could be prohibitive, and distortion of the lumenal geometry during deployment is a cause of concern. By providing an enhanced vertical adjustability, the physician could make adjustments *in situ,* and the devices could be fabricated in a reasonable number of standard lengths.

To provide such an enhanced adjustability, and also to maximize axial flexibility with adequate flow division and column strength, an alternative bifurcated module 310 has a minimized trunk length 312, as little as 1 cm. The smaller diameter branch portions provide axial flexibility to accommodate tortuosity, and the variable overlap region (here visible as gate 298) is at least about 2.5 cm, typically being over about 3.5 cm. The bifurcation septum 270 will be quite close to the end of the trunk lumen, optionally being even with the end of the trunk lumen, to avoid extending the inserted branch beyond the septum. The length of the integral first leg 262 is limited so that prosthesis length 316 will not occlude the hypogastric of the shortest renal-hypogastric distance anticipated for a given bifurcation module size. The length of the integral first leg can be increase by adding cuff modules as required. The position of the branch module is optionally further indicated by an imageable position scale 314. Peanut rings 292 maintain flow dynamics.

Although the exemplary embodiments have been described in some detail, by way of illustration and example, the scope of the present invention is limited solely by the appended claims.

## Claims

1. A branching endoluminal prosthesis (60) for use in a branching body lumen system which includes a trunk lumen and first and second branch lumens, the prosthesis comprising;
a radially expandable tubular trunk portion (64) having a prosthetic trunk lumen;
radially expandable tubular first and second branch portions (68) with first and second prosthetic branch lumens; and
a radially expandable tubular lumen separation portion (62) which provides fluid communication between the prosthetic trunk lumen and the first and second prosthetic branch lumens;
wherein the expanded trunk portion (64) is more axially flexible than the expanded lumen separation portion (62).

2. A branching endoluminal prosthesis as in Claim 1, wherein the prosthetic trunk lumen and the first and second prosthetic branch lumens adjacent the lumen separation portion define a branch plane, and wherein the trunk portion (64) has greater axial flexibility roughly perpendicular to the branch plane than the lumen separation portion (62).

3. A branching endoluminal prosthesis as in Claim 2, further comprising a trunk sealing cuff (66) on the trunk portion generally opposite the lumen separation to seal between the prosthetic trunk lumen and the trunk lumen of the body lumen system.

4. A branching endoluminal prosthesis as in Claim 3, wherein the trunk portion (64) is more axially flexible than the trunk sealing cuff.

5. A branching endoluminal prosthesis as in Claim 1, wherein at least a portion of the first and second branch portions (68) are more axially flexible than the lumen separation portion (62).

6. A branching endoluminal prosthesis as in Claim 5, further comprising branch sealing cuffs (70) on the first and second branch portions (68) generally opposite the lumen separation to seal between the prosthetic branch lumens and the branch lumens of the body lumen system.

7. A branching endoluminal prosthesis as in Claim 6, wherein the branch portions (68) are more axially flexible than the trunk sealing cuffs.

8. A branching endoluminal prosthesis as in Claim 1, wherein at least one of the trunk portion (64) and the first and second branch portions (68) comprises a liner supported by a helical coil (84) defining a plurality of separated loops to enhance axial flexibility, and wherein the helical coil (84) elongates during expansion of the liner to avoid unwinding of the coil (84) relative to the liner.

9. A branching endoluminal prosthesis according to any of claims 1 to 9 having a non-uniform structure, the axial flexibility of which is locally optimised to reduce distortion of the flow balance between the said branches (68) when conforming the prosthetic geometry to a tortuous body lumen system.

10. A branching endoluminal prosthesis according to any of claims 1 to 9 wherein the separation portion (62) comprises a relatively rigid structure having higher column and hoop strength than the remainder of the prosthesis (60).

## Patentansprüche

1. Endoluminale Zweigprothese (60) zur Verwendung in einem sich verzweigenden Körperlumensystem, das ein Haupt- bzw. Stammlumen und erste und zweite Zweiglumen enthält, wobei die Prothese umfaßt:
ein radial erweiterbares rohrförmiges Haupt- bzw. Stammlumenteil (64), das ein prothetisches Haupt- bzw. Stammlumen aufweist,
radial erweiterbare rohrförmige erste und zweite Zweiglumenteile (68) mit ersten und zweiten prothetischen Zweiglumen, und
ein radial erweiterbares rohrförmiges Haupt- bzw. Lumentrennteil (62), das eine Flüssigkeitsverbindung zwischen dem prothetischen Haupt- bzw. Stammlumen und dem ersten und dem zweiten prothetischen Zweiglumen schafft,
wobei das erweiterte Haupt- bzw. Stammteil (64) axial flexibler als das erweiterte Haupt- bzw. Stammtrennteil (62) ist.

2. Endoluminale Zweigprothese nach Anspruch 1, bei der das prothetische Haupt- bzw. Stammlumen und die ersten und zweiten prothetischen Zweiglumen benachbart zum Lumentrennteil eine Zweigebene definieren, und wobei das Haupt- bzw. Stammteil (64) eine größere axiale Flexibilität im wesentlichen rechtwinklig zur Zweigebene aufweist als das Lumentrennteil (62).

3. Endoluminale Zweigprothese nach Anspruch 2, umfassend eine Haupt- bzw. Stammdichtungsmanschette (66) auf dem Haupt- bzw. Stammteil im wesentlichen gegenüber der Lumentrennung, um zwischen dem prothetischen Haupt- bzw. Stammlumen und dem Haupt- bzw. Stammlumen des Körperlumensystems zu dichten.

4. Endoluminale Zweigprothese nach Anspruch 3, bei der das Haupt- bzw. Stammteil (64) axial flexibler als die Haupt- bzw. Stammdichtungsmanschette ist.

5. Endoluminale Zweigprothese nach Anspruch 1, bei der wenigstens ein Teil der ersten und zweiten Zweigteile (68) axial flexibler als das Lumentrennteil (62) ist.

6. Endoluminale Zweigprothese nach Anspruch 5, welche weiterhin Zweigdichtungsmanschetten (70) auf den ersten und zweiten Zweigteilen (68) im wesentlichen gegenüber der Lumentrennung aufweist, um zwischen den prothetischen Zweiglumen und den Zweiglumen des Körperlumensystems zu dichten.

7. Endoluminale Zweigprothese nach Anspruch 6, bei der die Haupt- bzw. Stammteile (68) axial flexibler als die Zweigdichtungsmanschetten sind.

8. Endoluminale Zweigprothese nach Anspruch 1, bei der wenigstens eines der Haupt- bzw. Stammteile (64) und die ersten und die zweiten Zweigteile (68) ein Futter, das durch eine helixförmige Wendel (84) aufgenommen wird, umfassen, wobei die Wendel (84) eine Mehrzahl von getrennten Windungen umfaßt, um die axiale Flexibilität zu erhöhen, und wobei die helixförmige Wendel (84) sich während der Ausdehnung des Futters verlängert, um ein Abwickeln der Wendel (84) relativ zum Futter zu vermeiden.

9. Endoluminare Zweigprothese nach einem der Ansprüche 1 bis 9, welche einen ungleichförmigen Aufbau aufweist, wobei deren axiale Flexibilität örtlich optimiert ist, um eine Zerstörung des Fließgleichgewichtes zwischen den Zweigen (68) zu vermindern, wenn die prothetische Geometrie an ein gewundenes Körperlumensystem angepaßt wird.

10. Endoluminare Zweigprothese nach einem der Ansprüche 1 bis 9, bei der das Trennteil (62) einen relativ festen Aufbau mit höherer Knick- und Umfangsfestigkeit als das übrige der Prothese (60) aufweist.

## Revendications

1. Prothèse endoluminale ramifiée (60) à utiliser dans un système ramifié de lumières du corps humain, comprenant une lumière de tronc et une première et une deuxième lumières branches, la prothèse comprenant:
une partie tubulaire formant tronc (64) pouvant se dilater radialement, ayant une lumière tronc prothétique;
une première et une deuxième parties tubulaires formant branches (68) pouvant se dilater radialement, avec une première et une deuxième lumières branches prothétiques; et
une partie tubulaire (62), pouvant se dilater radialement, de séparation de lumières, qui assure une communication de fluide entre la lumière tronc prothétique et les première et deuxième lumières branches prothétiques;
la partie dilatée formant tronc (64) étant plus souple, axialement, que la partie dilatée (62) de séparation de lumières.

2. Prothèse endoluminale ramifiée selon la revendication 1, dans laquelle la lumière tronc prothétique et les première et deuxième lumières branches prothétiques adjacentes à la partie de séparation de lumières définissent un plan de ramification, et dans laquelle la partie formant tronc (64) a, à peu près perpendiculairement au plan de ramification, une souplesse axiale plus grande que la partie de séparation (62) de lumières.

3. Prothèse endoluminale ramifiée selon la revendication 2, comprenant en outre, globalement à l'opposé de la séparation de lumières, un ballonnet (66) d'étanchéité de tronc sur la partie formant tronc pour assurer l'étanchéité entre la lumière tronc prothétique et la lumière tronc du système de lumières du corps humain.

4. Prothèse endoluminale ramifiée selon la revendication 3, dans laquelle la partie formant tronc (64) est plus souple, axialement, que le ballonnet d'étanchéité de tronc.

5. Prothèse endoluminale ramifiée selon la revendication 1, dans laquelle au moins une portion des première et deuxième parties formant branches (68) est, axialement, plus souple que la partie de séparation (62) de lumières.

6. Prothèse endoluminale ramifiée selon la revendication 5, comprenant en outre, globalement à l'opposé de la séparation de lumières, des ballonnets d'étanchéité (70) de branches sur les première et deuxième parties formant branches (68) pour assurer l'étanchéité entre les lumières branches prothétiques et les lumières branches du système de lumières du corps humain.

7. Prothèse endoluminale ramifiée selon la revendication 6, dans laquelle les parties formant branches (68) sont, axialement, plus souples que les ballonnets d'étanchéité de tronc.

8. Prothèse endoluminale ramifiée selon la revendication 1, dans laquelle au moins une des partie formant tronc (64) et première et deuxième parties formant branches (68) comporte une doublure supportée par un élément hélicoïdal (84) définissant plusieurs boucles séparées pour accroître la souplesse axiale, et dans laquelle l'élément hélicoïdal (84) s'allonge pendant la dilatation de la doublure pour éviter que l'élément hélicoïdal (84) ne se déroule par rapport à la doublure.

9. Prothèse endoluminale ramifiée selon l'une quelconque des revendications 1 à 9 ayant une structure non uniforme, dont la souplesse axiale est optimisée localement pour réduire la déformation de l'équilibre d'écoulement entre lesdites branches (68) en amenant la géométrie prothétique à épouser un système tortueux de lumières du corps humain.

10. Prothèse endoluminale ramifiée selon l'une quelconque des revendications 1 à 9, dans laquelle la partie de séparation (62) comprend une structure relativement rigide à résistance mécanique supérieure au reste de la prothèse (60) dans les directions verticale et circonférentielle.
